# EUROPEAN PATENT SPECIFICATION

(11) **EP 4 010 472 B1**
(45) Date of publication and mention of the grant of the patent: **01.01.2025**
(21) Application number: 20850363.1
(22) Date of filing: 05.08.2020
(51) Int. Cl.: C12N 15/10, C12N 15/11, C12Q 1/68, C12Q 1/6806, C12Q 1/6844, C12Q 1/686

(54) **METHOD AND APPARATUS FOR SINGLE-CELL ANALYSIS FOR DETERMINING A CELL TRAJECTORY**
VERFAHREN UND VORRICHTUNG ZUR EINZELZELLENANALYSE ZUR BESTIMMUNG EINER ZELLTRAJEKTORIE
PROCÉDÉ ET APPAREIL D'ANALYSE DE CELLULES UNIQUES POUR LA DÉTERMINATION D'UNE TRAJECTOIRE CELLULAIRE

(30) Priority: 05.08.2019 US 201962882750 P
(43) Date of publication of application: 15.06.2022
(73) Proprietor: Mission Bio, Inc., South San Francisco, California 94080 (US)
(72) Inventor: RUFF, David, South San Francisco, CA 94080 (US); DHINGRA, Dalia, South San Francisco, CA 94080 (US); OOI, Aik, South San Francisco, CA 94080 (US); MENDEZ, Pedro, South San Francisco, CA 94080 (US)
(74) Representative: Elkington and Fife LLP
(86) International application number: PCT/US2020/045001
(87) International publication number: WO 2021/026228

(56) References cited:
- WO-A1-2018/218226
- US-A1- 2015 132 743
- US-A1- 2015 337 298
- US-A1- 2016 032 282
- US-A1- 2018 216 160
- POTT ET AL.: "Simultaneous measurement of chromatin accessibility, DNA methylation, and nucleosome phasing in single cells", ELIFE, vol. 6, e23203, 27 July 2017 (2017-07-27), pages 1 - 19, XP055509996
- TSOMPANA ET AL.: "Chromatin accessibility: a window into the genome", EPIGENETICS CHROMATIN, vol. 7, no. 33, 20 November 2014 (2014-11-20), pages 1 - 16, XP021203650

## Description

This application claims the benefit of and priority to U.S. Provisional Patent Application No. 62/882,750 filed August 5, 2019.

### BACKGROUND

Single cell analysis has significantly advanced over the years, enabling the interrogation of cellular genomics, transcriptomics, and/or protein expression. Prior efforts have utilized parallel RNA sequencing and Assay for Transposase-Accessible Chromatin using sequencing (ATAC-seq) (e.g., see US Patent Application Publication No. 2019-0153436 A1, or WO-A-2018/218226 that describes methods and systems for sample preparation techniques that allow amplification e.g., whole genome amplification and sequencing of chromatin accessible regions of single cells). However, these workflow protocols require a variety of biological reagents, such as proteases and transposases (e.g., Tn5 transposase) for processing analytes in a cell. The inclusion of various biological reagents significantly complicates the workflow process and may lead to noisy and/or faulty sequence reads. Furthermore, the inclusion of various biological reagents for single-cell analysis is a costly endeavor, especially in scenarios where increasing numbers of cells from various specimen are to be analyzed.

### SUMMARY

The disclosure generally relates to methods and apparati for single-cell analysis through combined RNA sequencing and DNA sequencing of chromatin-accessible DNA. Here, the DNA sequencing workflow process represents an alternative to ATAC-seq. The DNA sequencing workflow minimizes or avoids the use of transposases, such as Tn5 transposase. Such methods involve a two-step workflow including a first step of encapsulating a cell within an emulsion and exposing the cell to reagents that cause the cell to lyse. In various embodiments, the reagents include detergents for lysing the cell, but minimizes or avoids the use of proteases, such as proteinase K. Such reagents that minimize or avoid using proteases or transposases are preferable because this simplifies the single-cell workflow process by requiring fewer consumables. Furthermore, this reduces the costs of consumables and operations of the single-cell workflow process, especially when analyzing large numbers of cells. The cell lysate includes RNA transcripts and packaged DNA (e.g., DNA packaged as chromatin). RNA transcripts are reverse transcribed to generate corresponding cDNA. The second step involves encapsulating at least the cDNA and packaged DNA into a second emulsion with barcodes and/or a reaction mixture. Within the second emulsion, the cDNA and packaged DNA undergo barcoding and the reaction mixture is used to perform a nucleic acid amplification reaction. Segments of DNA that are accessible (e.g., chromatin-accessible DNA) in the packaged DNA can be primed and amplified. Amplified nucleic acids are sequenced to generate sequence reads derived from the RNA transcripts and chromatin-accessible DNA. The generated sequence reads are analyzed to determine the trajectory of the single cell. For example, sequence reads of the RNA transcripts provide a snapshot of a prior state of the single cell whereas sequence reads of the chromatin-accessible DNA provide a snapshot of a future state of the single cell.

Disclosed herein is a method for predicting a cell trajectory for a cell, the method comprising: encapsulating a cell in an emulsion comprising reagents, the cell comprising at least one RNA molecule and packaged DNA comprising a segment of chromatin accessible-DNA; lysing the cell within the emulsion, thereby exposing the RNA and the packaged DNA to the reagents, wherein the reagents comprise less than 0.50 mg/mL protease and less than 2.5% (v/v) transposase; generating at least one cDNA molecule using the at least one RNA; encapsulating the at least one cDNA molecule, the packaged DNA, and a reaction mixture in a second emulsion; performing a nucleic acid amplification reaction within the second emulsion using the reaction mixture to generate a plurality of nucleic acids, the plurality of nucleic acids comprising: a first nucleic acid from one of the at least one cDNA molecule; and a second nucleic acid derived from the segment of chromatin-accessible DNA of the packaged DNA; and sequencing the first nucleic acid and the second nucleic acid. In various embodiments, the reagents comprise less than 0.10 mg/mL protease. In various embodiments, the reagents comprise less than 0.01 mg/mL protease. In various embodiments, the reagents do not include protease. In various embodiments, the reagents comprise less than 0.1% (v/v) transposase. In various embodiments, the reagents comprise less than 0.01% (v/v) transposase. In various embodiments, the reagents do not include transposase.

In various embodiments, performing the nucleic acid amplification reaction within the second emulsion using the reaction mixture to generate the plurality of nucleic acids comprises: priming the segment of the chromatin-accessible DNA in the packaged DNA; and generating an extended product from the primed segment of the chromatin-accessible DNA. In various embodiments, the method further comprises: in the emulsion, generating an extended product from a segment of the chromatin-accessible DNA in the packaged DNA, and wherein encapsulating the at least one cDNA molecule, the packaged DNA, and a reaction mixture in the second emulsion further comprises encapsulating the extended product in the second emulsion. In various embodiments, generating the extended product from a segment of the chromatin-accessible DNA in the packaged DNA comprises: exposing the first emulsion to a temperature between 40°C and 60°C, thereby destabilizing the segment of the chromatin-accessible DNA.

In various embodiments, the reagents comprise reverse transcriptase. In various embodiments, the reagents comprise NP-40. In various embodiments, the method further comprises predicting the cell trajectory using the sequenced first nucleic acid and the sequenced second nucleic acid. In various embodiments, predicting the cell trajectory comprises using at least the sequenced first nucleic acid and second nucleic acid to determine two different states of the cell. In various embodiments, the sequenced first nucleic acid is used to determine a prior state of the cell and wherein the sequenced second nucleic acid is used to determine a future state of the cell.

In various embodiments, the at least one RNA is previously transcribed from a DNA region that comprises one chromatin-accessible DNA, thereby indicating a commonality between the prior state and future state of the cell. In various embodiments, the at least one RNA is transcribed from a DNA region that corresponds to chromatininaccessible DNA, thereby indicating a transition from the prior state of the cell towards the future state of the cell. In various embodiments, the cell trajectory is any one of a cell lineage, a cell fate, a cell function in a future state of the cell, a diseased future state of the cell, or a future cellular response to an external stimulus.

In various embodiments, the method further comprises encapsulating a first barcode and a second barcode in the second emulsion along with the at least one cDNA, at least one chromatin-accessible DNA, and the reaction mixture. In various embodiments, the first nucleic acid comprises the first barcode. In various embodiments, the second nucleic acid comprises the second barcode. In various embodiments, the first barcode and second barcode share a same barcode sequence. In various embodiments, the first barcode and second barcode share different barcode sequences. In various embodiments, the first barcode and second barcode are releasably attached to a bead in the second emulsion.

In various embodiments, reverse transcribing the at least one RNA occurs within the first emulsion. In various embodiments, the nucleic acid amplification reaction is polymerase chain reaction. In various embodiments, wherein the plurality of nucleic acids further comprise nucleic acids derived from other segments of chromatin-accessible DNA in the packaged DNA corresponding to intronic DNA regions. In various embodiments, at least 50% of the nucleic acids derived from other chromatin-accessible DNA molecules of the packaged DNA corresponding to intronic DNA regions are between 100 to 500 base pairs in length.

Additionally disclosed herein is a system comprising: a device configured to: encapsulate a cell in an emulsion comprising reagents, the cell comprising at least one RNA molecule and packaged DNA comprising a segment of chromatin accessible-DNA; lyse the cell within the emulsion, thereby exposing the RNA and the packaged DNA to the reagents, wherein the reagents comprise less than 0.50 mg/mL protease and less than 2.5% (v/v) transposase; generate at least one cDNA molecule by reverse transcribing the at least one RNA; and encapsulate the at least one cDNA molecule, the packaged DNA, and reagents in a second emulsion; perform a PCR reaction within the second emulsion to generate a plurality of nucleic acids, the plurality of nucleic acids comprising: a first nucleic acid from one of the at least one cDNA molecule; and a second nucleic acid derived from the segment of chromatin-accessible DNA of the packaged DNA; and sequence the first nucleic acid and the second nucleic acid.

In various embodiments, the system further comprises: a computational device communicatively coupled to the device, the computational device configured to predict the cell trajectory by using the sequenced first nucleic acid and the second nucleic acid.

In various embodiments, the reagents comprise less than 0.10 mg/mL protease. In various embodiments, the reagents comprise less than 0.01 mg/mL protease. In various embodiments, the reagents do not include protease. In various embodiments, the reagents comprise less than 0.1% (v/v) transposase. In various embodiments, the reagents comprise less than 0.01% (v/v) transposase. In various embodiments, the reagents do not include transposase.

In various embodiments, performing the nucleic acid amplification reaction within the second emulsion using the reaction mixture to generate the plurality of nucleic acids comprises: priming the segment of the chromatin-accessible DNA in the packaged DNA; and generating an extended product from the primed segment of the chromatin-accessible DNA.

In various embodiments, the system is further configured to: in the emulsion, generate an extended product from a segment of the chromatin-accessible DNA in the packaged DNA, and wherein encapsulating the at least one cDNA molecule, the packaged DNA, and a reaction mixture in the second emulsion further comprises encapsulating the extended product in the second emulsion. In various embodiments, generating the extended product from a segment of the chromatin-accessible DNA in the packaged DNA comprises: exposing the first emulsion to a temperature between 40°C and 60°C, thereby destabilizing the segment of the chromatin-accessible DNA.

In various embodiments, the reagents comprise reverse transcriptase. In various embodiments, the reagents comprise NP-40. In various embodiments, predicting the cell trajectory comprises using at least the sequenced first nucleic acid and second nucleic acid to determine two different states of the cell. In various embodiments, the sequenced first nucleic acid is used to determine a prior state of the cell and wherein the sequenced second nucleic acid is used to determine a future state of the cell.

In various embodiments, the at least one RNA is previously transcribed from a DNA region that comprises one chromatin-accessible DNA, thereby indicating a commonality between the prior state and future state of the cell. In various embodiments, the at least one RNA is transcribed from a DNA region that corresponds to chromatininaccessible DNA, thereby indicating a transition from the prior state of the cell towards the future state of the cell. In various embodiments, the cell trajectory is any one of a cell lineage, a cell fate, a cell function in a future state of the cell, a diseased future state of the cell, or a future cellular response to an external stimulus. In various embodiments, the device is further configured to encapsulate a first barcode and a second barcode in the second emulsion along with the at least one cDNA, at least one chromatin-accessible DNA, and the reaction mixture.

In various embodiments, the first nucleic acid comprises the first barcode. In various embodiments, the second nucleic acid comprises the second barcode. In various embodiments, the first barcode and second barcode share a same barcode sequence. In various embodiments, the first barcode and second barcode share different barcode sequences. In various embodiments, the first barcode and second barcode are releasably attached to a bead in the second emulsion. In various embodiments, reverse transcribing the at least one RNA occurs within the first emulsion. In various embodiments, the nucleic acid amplification reaction is polymerase chain reaction.

In various embodiments, the plurality of nucleic acids further comprise nucleic acids derived from other segments of chromatin-accessible DNA in the packaged DNA corresponding to intronic DNA regions. In various embodiments, at least 50% of the nucleic acids derived from other chromatin-accessible DNA molecules of the packaged DNA corresponding to intronic DNA regions are between 100 to 500 base pairs in length.

### BRIEF DESCRIPTION OF THE SEVERAL VIEWS OF THE DRAWINGS

These and other features, aspects, and advantages of the present invention will become better understood with regard to the following description, and accompanying drawings, where:
Figure (FIG.) 1 shows an embodiment of processing single cells to generate amplified nucleic acid molecules for sequencing.
FIG. 2 is a flow process for determining a cell trajectory of a single cell using sequencing reads derived from analytes of the single cell.
FIGs. 3A-3C depict the processing and releasing analytes of a single cell in an emulsion, in accordance with an embodiment that does not include the use of proteases or transposases.
FIG. 4A depicts the processing of RNA and packaged DNA in a first emulsion, in accordance with a first embodiment.
FIG. 4B depicts the amplification and barcoding of nucleic acids derived from RNA and chromatin accessible DNA, in accordance with the first embodiment shown in FIG. 4A.
FIG. 4C depicts the processing of RNA and packaged DNA in a first emulsion, in accordance with a second embodiment.
FIG. 4D depicts the amplification and barcoding of nucleic acids derived from RNA and chromatin accessible DNA, in accordance with the second embodiment shown in FIG. 4C.
FIG. 5A depicts sequencing reads obtained through single-cell RNA-seq and DNA-seq for determining a cell trajectory, in accordance with a first embodiment.
FIG. 5B depicts sequencing reads obtained through single-cell RNA-seq and DNA-seq for determining a cell trajectory, in accordance with a second embodiment.
FIG. 6 depicts an overall system environment including a single cell workflow device and a computational device for conducting single-cell analysis to predict cell trajectories.
FIG. 7 depicts an example computing device for implementing system and methods described in reference to FIGs. 1-6.
FIG. 8A depicts DNA amplicon sizes observed with reads in intronic regions obtained through oligo dT priming of K-562 cells, where no proteinase K and no transposase (Tn5) was used during encapsulation.
FIGs. 8B and 8C show integrative genomics viewer (IGV) screenshots of sequence reads aligned to the reference genome (aligned to the CCL2 gene and HLA-C gene, respectively).
FIG. 9A depicts DNA amplicon sizes observed with reads in intronic regions obtained through gene specific priming of MCF7 cells, where no proteinase K and no transposase (Tn5) was used during encapsulation.
FIGs. 9B and 9C show integrative genomics viewer (IGV) screenshots of sequence reads aligned to the reference genome (aligned to the VIM gene and MKI67 gene, respectively).

### DETAILED DESCRIPTION

### Definitions

Terms used in the claims and specification are defined as set forth below unless otherwise specified.

The term "subject" or "patient" are used interchangeably and encompass an organism, human or non-human, mammal or non-mammal, male or female.

The term "sample" or "test sample" can include a single cell or multiple cells or fragments of cells or an aliquot of body fluid, such as a blood sample, taken from a subject, by means including venipuncture, excretion, ejaculation, massage, biopsy, needle aspirate, lavage sample, scraping, surgical incision, or intervention or other means known in the art.

The term "analyte" refers to a component of a cell. Cell analytes can be informative for understanding a state, behavior, or trajectory of a cell. Therefore, performing single-cell analysis of one or more analytes of a cell using the systems and methods described herein are informative for determining a state or behavior of a cell. Examples of an analyte include a nucleic acid (e.g., RNA, DNA, cDNA), a protein, a peptide, an antibody, an antibody fragment, a polysaccharide, a sugar, a lipid, a small molecule, or combinations thereof. In particular embodiments, a single-cell analysis involves analyzing two different analytes such as RNA and DNA. In particular embodiments, a single-cell analysis involves analyzing three or more different analytes of a cell, such as RNA, DNA, and protein.

In some embodiments, the discrete entities as described herein are droplets. The terms "emulsion," "drop," "droplet," and "microdroplet" are used interchangeably herein, to refer to small, generally spherically structures, containing at least a first fluid phase, e.g., an aqueous phase (e.g., water), bounded by a second fluid phase (e.g., oil) which is immiscible with the first fluid phase. In some embodiments, droplets according to the present disclosure may contain a first fluid phase, e.g., oil, bounded by a second immiscible fluid phase, e.g. an aqueous phase fluid (e.g., water). In some embodiments, the second fluid phase will be an immiscible phase carrier fluid. Thus droplets according to the present disclosure may be provided as aqueous-in-oil emulsions or oil-in-aqueous emulsions. Droplets may be sized and/or shaped as described herein for discrete entities. For example, droplets according to the present disclosure generally range from 1 µm to 1000 µm, inclusive, in diameter. Droplets according to the present disclosure may be used to encapsulate cells, nucleic acids (e.g., DNA), enzymes, reagents, reaction mixture, and a variety of other components. The term emulsion may be used to refer to an emulsion produced in, on, or by a microfluidic device and/or flowed from or applied by a microfluidic device.

The term "cell trajectory" or "trajectory of a cell" refers to a change of a cell from a first state to a second state. A "cell trajectory" is determined through a single-cell analysis that combines RNA-seq and DNA-seq (e.g., sequencing of chromatin-accessible DNA). Sequencing reads obtained through RNA-seq provide a snapshot of a past state of the cell whereas sequencing reads obtained through DNA-seq provide a snapshot of a future state of the cell. Examples of a cell trajectory include any of a cell lineage, a cell fate, a cell function in a future state of the cell, a diseased future state of the cell, a future cellular response to an external stimulus (e.g., a treatment).

"Complementarity" refers to the ability of a nucleic acid to form hydrogen bond(s) or hybridize with another nucleic acid sequence by either traditional Watson-Crick or other non-traditional types. As used herein "hybridization," refers to the binding, duplexing, or hybridizing of a molecule only to a particular nucleotide sequence under low, medium, or highly stringent conditions, including when that sequence is present in a complex mixture (e.g., total cellular) DNA or RNA. See e.g. Ausubel, et al., Current Protocols In Molecular Biology, John Wiley & Sons, New York, N.Y., 1993. If a nucleotide at a certain position of a polynucleotide is capable of forming a Watson-Crick pairing with a nucleotide at the same position in an anti-parallel DNA or RNA strand, then the polynucleotide and the DNA or RNA molecule are complementary to each other at that position. The polynucleotide and the DNA or RNA molecule are "substantially complementary" to each other when a sufficient number of corresponding positions in each molecule are occupied by nucleotides that can hybridize or anneal with each other in order to affect the desired process. A complementary sequence is a sequence capable of annealing under stringent conditions to provide a 3'-terminal serving as the origin of synthesis of complementary chain.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, Md.). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research 12(1): 387 (1984)), BLASTP, BLASTN, and FASTA (Atschul, S. F. et al., J. Molec. Biol. 215:403-410 (1990)). The BLAST X program is publicly available from NCBI and other sources (BLAST Manual, Altschul, S., et al., NCBINLM NIH Bethesda, Md. 20894: Altschul, S., et al., J. Mol. Biol. 215:403-410 (1990). The well-known Smith Waterman algorithm may also be used to determine identity.

The terms "amplify," "amplifying," "amplification reaction" and their variants, refer generally to any action or process whereby at least a portion of a nucleic acid molecule (referred to as a template nucleic acid molecule) is replicated or copied into at least one additional nucleic acid molecule. The additional nucleic acid molecule optionally includes sequence that is substantially identical or substantially complementary to at least some portion of the template nucleic acid molecule. The template nucleic acid molecule can be single-stranded or double-stranded and the additional nucleic acid molecule can independently be single-stranded or double-stranded. In some embodiments, amplification includes a template-dependent in vitro enzyme-catalyzed reaction for the production of at least one copy of at least some portion of the nucleic acid molecule or the production of at least one copy of a nucleic acid sequence that is complementary to at least some portion of the nucleic acid molecule. Amplification optionally includes linear or exponential replication of a nucleic acid molecule. In some embodiments, such amplification is performed using isothermal conditions; in other embodiments, such amplification can include thermocycling. In some embodiments, the amplification is a multiplex amplification that includes the simultaneous amplification of a plurality of target sequences in a single amplification reaction. At least some of the target sequences can be situated, on the same nucleic acid molecule or on different target nucleic acid molecules included in the single amplification reaction. In some embodiments, "amplification" includes amplification of at least some portion of DNA- and RNA-based nucleic acids alone, or in combination. The amplification reaction can include single or double-stranded nucleic acid substrates and can further include any of the amplification processes known to one of ordinary skill in the art. In some embodiments, the amplification reaction includes polymerase chain reaction (PCR). In some embodiments, the amplification reaction includes an isothermal amplification reaction such as LAMP. In the present invention, the terms "synthesis" and "amplification" of nucleic acid are used. The synthesis of nucleic acid in the present invention means the elongation or extension of nucleic acid from an oligonucleotide serving as the origin of synthesis. If not only this synthesis but also the formation of other nucleic acid and the elongation or extension reaction of this formed nucleic acid occur continuously, a series of these reactions is comprehensively called amplification. The polynucleic acid produced by the amplification technology employed is generically referred to as an "amplicon" or "amplification product."

Any nucleic acid amplification method may be utilized, such as a PCR-based assay, e.g., quantitative PCR (qPCR), or an isothermal amplification may be used to detect the presence of certain nucleic acids, e.g., genes of interest, present in discrete entities or one or more components thereof, e.g., cells encapsulated therein. Such assays can be applied to discrete entities within a microfluidic device or a portion thereof or any other suitable location. The conditions of such amplification or PCR-based assays may include detecting nucleic acid amplification over time and may vary in one or more ways.

A number of nucleic acid polymerases can be used in the amplification reactions utilized in certain embodiments provided herein, including any enzyme that can catalyze the polymerization of nucleotides (including analogs thereof) into a nucleic acid strand. Such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also includes fusion proteins comprising at least two portions linked to each other, where the first portion comprises a peptide that can catalyze the polymerization of nucleotides into a nucleic acid strand and is linked to a second portion that comprises a second polypeptide. In some embodiments, the second polypeptide can include a reporter enzyme or a processivity-enhancing domain. Optionally, the polymerase can possess 5' exonuclease activity or terminal transferase activity. In some embodiments, the polymerase can be optionally reactivated, for example through the use of heat, chemicals or re-addition of new amounts of polymerase into a reaction mixture. In some embodiments, the polymerase can include a hot-start polymerase or an aptamer-based polymerase that optionally can be reactivated.

The terms "target primer" or "target-specific primer" and variations thereof refer to primers that are complementary to a binding site sequence. Target primers are generally a single stranded or double- stranded polynucleotide, typically an oligonucleotide, that includes at least one sequence that is at least partially complementary to a target nucleic acid sequence.

"Forward primer binding site" and "reverse primer binding site" refers to the regions on the template DNA and/or the amplicon to which the forward and reverse primers bind. The primers act to delimit the region of the original template polynucleotide which is exponentially amplified during amplification. In some embodiments, additional primers may bind to the region 5' of the forward primer and/or reverse primers. Where such additional primers are used, the forward primer binding site and/or the reverse primer binding site may encompass the binding regions of these additional primers as well as the binding regions of the primers themselves. For example, in some embodiments, the method may use one or more additional primers which bind to a region that lies 5' of the forward and/or reverse primer binding region. Such a method was disclosed, for example, in WO0028082 which discloses the use of "displacement primers" or "outer primers".

A "barcode" nucleic acid identification sequence can be incorporated into a nucleic acid primer or linked to a primer to enable independent sequencing and identification to be associated with one another via a barcode which relates information and identification that originated from molecules that existed within the same sample. There are numerous techniques that can be used to attach barcodes to the nucleic acids within a discrete entity. For example, the target nucleic acids may or may not be first amplified and fragmented into shorter pieces. The molecules can be combined with discrete entities, e.g., droplets, containing the barcodes. The barcodes can then be attached to the molecules using, for example, splicing by overlap extension. In this approach, the initial target molecules can have "adaptor" sequences added, which are molecules of a known sequence to which primers can be synthesized. When combined with the barcodes, primers can be used that are complementary to the adaptor sequences and the barcode sequences, such that the product amplicons of both target nucleic acids and barcodes can anneal to one another and, via an extension reaction such as DNA polymerization, be extended onto one another, generating a double- stranded product including the target nucleic acids attached to the barcode sequence. Alternatively, the primers that amplify that target can themselves be barcoded so that, upon annealing and extending onto the target, the amplicon produced has the barcode sequence incorporated into it. This can be applied with a number of amplification strategies, including specific amplification with PCR or non-specific amplification with, for example, MDA. An alternative enzymatic reaction that can be used to attach barcodes to nucleic acids is ligation, including blunt or sticky end ligation. In this approach, the DNA barcodes are incubated with the nucleic acid targets and ligase enzyme, resulting in the ligation of the barcode to the targets. The ends of the nucleic acids can be modified as needed for ligation by a number of techniques, including by using adaptors introduced with ligase or fragments to enable greater control over the number of barcodes added to the end of the molecule.

The terms "identity" and "identical" and their variants, as used herein, when used in reference to two or more sequences, refer to the degree to which the two or more sequences (e.g., nucleotide or polypeptide sequences) are the same. In the context of two or more sequences, the percent identity or homology of the sequences or subsequences thereof indicates the percentage of all monomeric units (e.g., nucleotides or amino acids) that are the same at a given position or region of the sequence (i.e., about 70% identity, preferably 75%, 80%, 85%, 90%, 95%, 97%, 98% or 99% identity). The percent identity canbe over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

The terms "nucleic acid," "polynucleotides," and "oligonucleotides" refers to biopolymers of nucleotides and, unless the context indicates otherwise, includes modified and unmodified nucleotides, and both DNA and RNA, and modified nucleic acid backbones. For example, in certain embodiments, the nucleic acid is a peptide nucleic acid (PNA) or a locked nucleic acid (LNA). Typically, the methods as described herein are performed using DNA as the nucleic acid template for amplification. However, nucleic acid whose nucleotide is replaced by an artificial derivative or modified nucleic acid from natural DNA or RNA is also included in the nucleic acid of the present invention insofar as it functions as a template for synthesis of complementary chain. The nucleic acid of the present invention is generally contained in a biological sample. The biological sample includes animal, plant or microbial tissues, cells, cultures and excretions, or extracts therefrom. In certain aspects, the biological sample includes intracellular parasitic genomic DNA or RNA such as virus or mycoplasma. The nucleic acid may be derived from nucleic acid contained in said biological sample. For example, genomic DNA, or cDNA synthesized from mRNA, or nucleic acid amplified on the basis of nucleic acid derived from the biological sample, are preferably used in the described methods. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes deoxythymidine, and "U' denotes uridine. Oligonucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

A template nucleic acid is a nucleic acid serving as a template for synthesizing a complementary chain in a nucleic acid amplification technique. A complementary chain having a nucleotide sequence complementary to the template has a meaning as a chain corresponding to the template, but the relationship between the two is merely relative. That is, according to the methods described herein a chain synthesized as the complementary chain can function again as a template. That is, the complementary chain can become a template. In certain embodiments, the template is derived from a biological sample, e.g., plant, animal, virus, micro-organism, bacteria, fungus, etc. In certain embodiments, the animal is a mammal, e.g., a human patient. A template nucleic acid typically comprises one or more target nucleic acid. A target nucleic acid in exemplary embodiments may comprise any single or double-stranded nucleic acid sequence that can be amplified or synthesized according to the disclosure, including any nucleic acid sequence suspected or expected to be present in a sample.

Primers and oligonucleotides used in embodiments herein comprise nucleotides. A nucleotide comprises any compound, including without limitation any naturally occurring nucleotide or analog thereof, which can bind selectively to, or can be polymerized by, a polymerase. Typically, but not necessarily, selective binding of the nucleotide to the polymerase is followed by polymerization of the nucleotide into a nucleic acid strand by the polymerase; occasionally however the nucleotide may dissociate from the polymerase without becoming incorporated into the nucleic acid strand, an event referred to herein as a "non-productive" event. Such nucleotides include not only naturally occurring nucleotides but also any analogs, regardless of their structure, that can bind selectively to, or can be polymerized by, a polymerase. While naturally occurring nucleotides typically comprise base, sugar and phosphate moieties, the nucleotides of the present disclosure can include compounds lacking any one, some or all of such moieties. For example, the nucleotide can optionally include a chain of phosphorus atoms comprising three, four, five, six, seven, eight, nine, ten or more phosphorus atoms. In some embodiments, the phosphorus chain can be attached to any carbon of a sugar ring, such as the 5' carbon. The phosphorus chain can be linked to the sugar with an intervening O or S. In one embodiment, one or more phosphorus atoms in the chain can be part of a phosphate group having P and O. In another embodiment, the phosphorus atoms in the chain can be linked together with intervening O, NH, S, methylene, substituted methylene, ethylene, substituted ethylene, CNH₂, C(O), C(CH₂), CH₂CH₂, or C(OH)CH₂R (where R can be a 4-pyridine or 1-imidazole). In one embodiment, the phosphorus atoms in the chain can have side groups having O, BH3, or S. In the phosphorus chain, a phosphorus atom with a side group other than O can be a substituted phosphate group. In the phosphorus chain, phosphorus atoms with an intervening atom other than O can be a substituted phosphate group. Some examples of nucleotide analogs are described in Xu, U.S. Pat. No. 7,405,281.

In some embodiments, the nucleotide comprises a label and referred to herein as a "labeled nucleotide"; the label of the labeled nucleotide is referred to herein as a "nucleotide label." In some embodiments, the label can be in the form of a fluorescent moiety (e.g. dye), luminescent moiety, or the like attached to the terminal phosphate group, i.e., the phosphate group most distal from the sugar. Some examples of nucleotides that can be used in the disclosed methods and compositions include, but are not limited to, ribonucleotides, deoxyribonucleotides, modified ribonucleotides, modified deoxyribonucleotides, ribonucleotide polyphosphates, deoxyribonucleotide polyphosphates, modified ribonucleotide polyphosphates, modified deoxyribonucleotide polyphosphates, peptide nucleotides, modified peptide nucleotides, metallonucleosides, phosphonate nucleosides, and modified phosphate-sugar backbone nucleotides, analogs, derivatives, or variants of the foregoing compounds, and the like. In some embodiments, the nucleotide can comprise non-oxygen moieties such as, for example, thio- or boranomoieties, in place of the oxygen moiety bridging the alpha phosphate and the sugar of the nucleotide, or the alpha and beta phosphates of the nucleotide, or the beta and gamma phosphates of the nucleotide, or between any other two phosphates of the nucleotide, or any combination thereof. "Nucleotide 5'- triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group can include sulfur substitutions for the various oxygens, e.g. α-thio- nucleotide 5'-triphosphates. For a review of nucleic acid chemistry, see: Shabarova, Z. and Bogdanov, A. Advanced Organic Chemistry of Nucleic Acids, VCH, New York, 1994.

### Overview

Described herein are embodiments for performing single-cell analysis using combined RNA-seq and DNA-seq (e.g., DNA-seq of chromatin-accessible DNA) to predict a trajectory of the single cell. Examples of a cell trajectory include any of a cell lineage, a cell fate, a cell function in a future state of the cell, a diseased future state of the cell, a future cellular response to an external stimulus (e.g., a treatment). Generally, the single-cell analysis involves a workflow for processing single cells and performing sequencing (e.g., RNA-seq, DNA-seq, or both RNA-seq and DNA-seq) to obtain sequencing reads of analytes of the single cells. The single-cell analysis further includes *in silico* steps of analyzing the sequencing reads to determine the trajectory of the single cells.

In various embodiments, the workflow for processing a single cell enables the sequencing of nucleic acids derived from RNA transcripts in the single cell as well as the sequencing of nucleic acids derived from DNA that is accessible even when packaged with nucleosomes and chromatin (e.g., chromatin-accessible DNA). In various embodiments, a cell is exposed to reagents that include reverse transcriptase (for performing reverse transcription on RNA transcripts) but minimizes or avoids the use of proteases or transposases. Thus, the packaged DNA remains intact. RNA-seq can be performed to obtain sequencing reads of nucleic acid molecules derived from the RNA transcripts and DNA-seq can be performed to obtain sequencing reads of nucleic acid molecules derived from chromatin-accessible DNA. The sequencing reads obtained from RNA-seq and DNA-seq are analyzed to determine trajectories of individual cells.

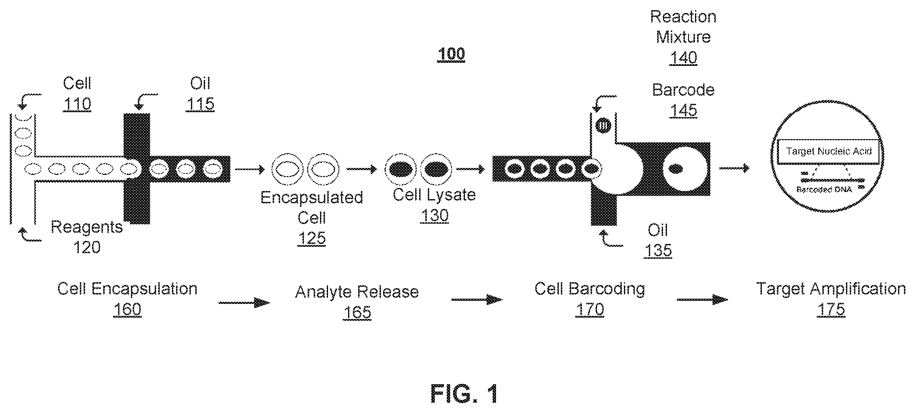

Reference is now made to FIG. 1, which depicts one embodiment of processing single cells to generate amplified nucleic acid molecules for sequencing. Specifically, FIG. 1 depicts a workflow process including the steps of cell encapsulation 160, analyte release 165, cell barcoding, and target amplification 175 of target nucleic acid molecules.

Generally, the cell encapsulation step 160 involves encapsulating a single cell 110 with reagents 120 into an emulsion. In various embodiments, the emulsion is formed by partitioning aqueous fluid containing the cell 110 and reagents 120 into a carrier fluid (e.g., oil 115), thereby resulting in a aqueous fluid-in-oil emulsion. The emulsion includes encapsulated cell 125 and the reagents 120. The encapsulated cell undergoes an analyte release at step 165. Generally, the reagents cause the cell to lyse, thereby generating a cell lysate 130 within the emulsion. In particular embodiments, the reagents 120 include at least reverse transcriptase. In various embodiments, the reagents 120 include reduced amounts of proteases or transposases or do not include proteases or transposases. For example, the reagents 120 include reduced amounts of proteinase K or do not include proteinase K (or mutated variants thereof) and further include reduced amounts of transposase Tn5 or do not include transposase Tn5 (or mutated variants thereof). The cell lysate 130 includes the contents of the cell, which can include one or more different types of analytes (e.g., RNA transcripts, DNA, protein, lipids, or carbohydrates). In various embodiments, the different analytes of the cell lysate 130 can interact with reagents 120 within the emulsion. For example, reverse transcriptase in the reagents 120 can reverse transcribe cDNA molecules from RNA transcripts that are present in the cell lysate 130.

The cell barcoding step 170 involves encapsulating the cell lysate 130 into a second emulsion along with a barcode 145 and/or reaction mixture 140. In various embodiments, the second emulsion is formed by partitioning aqueous fluid containing the cell lysate 130 into immiscible oil 135. As shown in FIG. 1, the reaction mixture 140 and barcode 145 can be introduced through a separate stream of aqueous fluid, thereby partitioning the reaction mixture 140 and barcode into the second emulsion along with the cell lysate 130.

Generally, a barcode 145 can label a target nucleic acid to be analyzed (e.g., an analyte of the cell lysate), which enables subsequent identification of the origin of a sequence read that is derived from the target nucleic acid. In various embodiments, multiple barcodes 145 can label multiple target nucleic acid of the cell lysate, thereby enabling the subsequent identification of the origin of large quantities of sequence reads. Generally, the reaction mixture 140 enables the performance of a reaction, such as a nucleic acid amplification reaction.

The target amplification step 175 involves amplifying target nucleic acids. For example, target nucleic acids of the cell lysate undergo amplification using the reaction mixture 140 in the second emulsion, thereby generating amplicons derived from the target nucleic acids. Although FIG. 1B depicts cell barcoding 170 and target amplification 175 as two separate steps, in various embodiments, the target nucleic acid is labeled with a barcode 145 through the nucleic acid amplification step.

As referred herein, the workflow process shown in FIG. 1 is a two-step workflow process in which analyte release 165 from the cell occurs separate from the steps of cell barcoding 170 and target amplification 175. For example, analyte release 165 from a cell occurs within a first emulsion followed by cell barcoding 170 and target amplification 175 in a second emulsion. In various embodiments, alternative workflow processes (e.g., workflow processes other than the two-step workflow process shown in FIG. 1) can be employed. For example, the cell 110, reagents 120, reaction mixture 140, and barcode 145 can be encapsulated in an emulsion. Thus, analyte release 165 can occur within the emulsion, followed by cell barcoding 170 and target amplification 175 within the same emulsion.

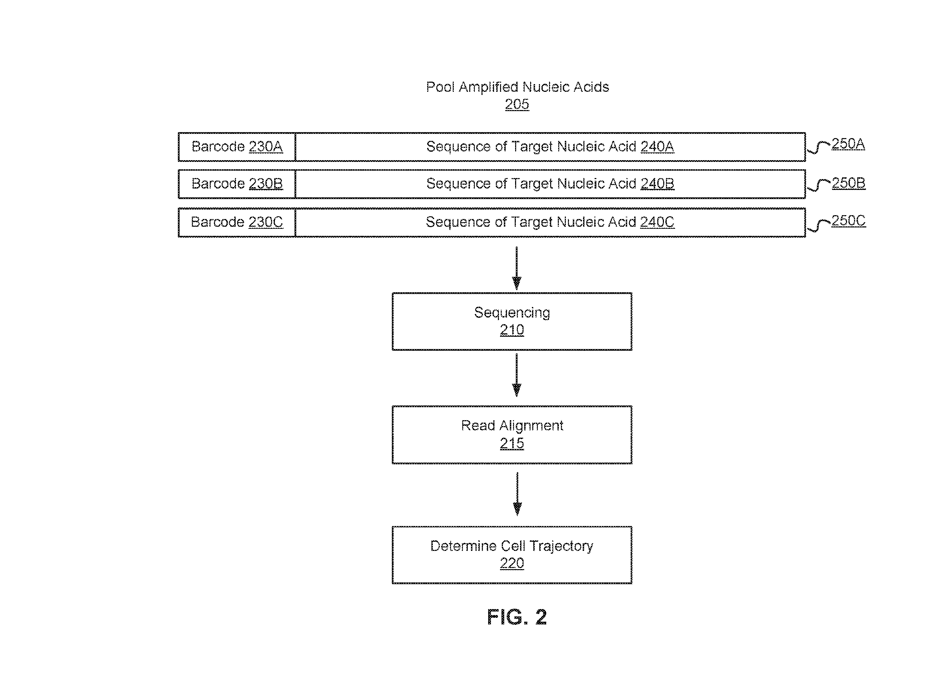

FIG. 2 is a flow process for determining a cell trajectory of a single cell using sequencing reads derived from analytes of the single cell. Specifically, FIG. 2 depicts the steps of pooling amplified nucleic acids at step 205, sequencing the amplified nucleic acids at step 210, read alignment at step 215, and determining a cell trajectory for a cell using the aligned sequence reads. Generally, the flow process shown in FIG. 2 is a continuation of the workflow process shown in FIG. 1.

For example, after target amplification at step 175 of FIG. 1, the amplified nucleic acids 250A, 250B, and 250C are pooled at step 205 shown in FIG. 2. For example, emulsions of amplified nucleic acids are pooled and collected, and the immiscible oil of the emulsions is removed. Thus, amplified nucleic acids from multiple cells can be pooled together. FIG. 2 depicts three amplified nucleic acids 250A, 250B, and 250C but in various embodiments, pooled nucleic acids can include hundreds, thousands, or millions of nucleic acids derived from analytes of multiple cells.

In various embodiments, each amplified nucleic acid 250 includes at least a sequence of a target nucleic acid 240 and a barcode 230. In various embodiments, an amplified nucleic acid 250 can include additional sequences, such as any of a universal primer sequence (e.g., an oligo-dT sequence), a random primer sequence, a gene specific primer forward sequence, a gene specific primer reverse sequence, or a constant region.

In various embodiments, the amplified nucleic acids 250A, 250B, and 250C are derived from the same single cell and therefore, the barcodes 230A, 230B, and 230C are the same. Therefore, sequencing of the barcodes 230 enables the determination that the amplified nucleic acids 250 are derived from the same cell. In various embodiments, the amplified nucleic acids 250A, 250B, and 250C are pooled and derived from different cells. Therefore, the barcodes 230A, 230B, and 230C are different from one another and sequencing of the barcodes 230 enables the determination that the amplified nucleic acids 250 are derived from different cells.

At step 210, the pooled amplified nucleic acids 250 undergo sequencing to generate sequence reads. For each amplified nucleic acid, the sequence read includes the sequence of the barcode and the target nucleic acid. Sequence reads originating from individual cells are clustered according to the barcode sequences included in the amplified nucleic acids. At step 215, the sequence reads for each single cell are aligned (e.g., to a reference genome). Aligning the sequence reads to the reference genome enables the determination of where in the genome the sequence read is derived from. For example, multiple sequence reads generated from a RNA transcript, when aligned to a position of the genome, can reveal that a gene at the position of the genome was transcribed. As another example, multiple sequence reads generated from chromatin-accessible DNA, when aligned to a position of the genome, can reveal that a gene at the position of the genome is accessible and can be transcribed.

At step 200, aligned sequence reads for a single cell are analyzed to determine the trajectory of the single cell. For example, sequence reads generated from RNA transcripts provide a snapshot of gene expression an earlier state of the cell. Additionally, sequence reads generated from chromatin-accessible DNA provide a snapshot of gene expression in a future state of the cell. Taken together, the earlier state of the cell and future state of the cell can be useful for determining the cell trajectory, such as any of a cell lineage, a cell fate, a cell function in a future state of the cell, a diseased future state of the cell, a future cellular response to an external stimulus (e.g., a treatment).

### Methods for Performing Single-Cell Analysis

### Encapuslation, Analyte Release, Barcoding, and Amplification

Embodiments described herein involve encapsulating one or more cells (e.g., at step 160 in FIG. 1) to perform single-cell analysis on the one or more cells. In various embodiments, the one or more cells can be isolated from a test sample obtained from a subject or a patient. In various embodiments, the one or more cells are healthy cells taken from a healthy subject. In various embodiments, the one or more cells include cancer cells taken from a subject previously diagnosed with cancer. For example, such cancer cells can be tumor cells available in the bloodstream of the subject diagnosed with cancer. Thus, single-cell analysis of the tumor cells enables cellular and sub-cellular prediction of the subject's cancer. In various embodiments, the test sample is obtained from a subject following treatment of the subject (e.g., following a therapy such as cancer therapy). Thus, single-cell analysis of the cells enables cellular and sub-cellular prediction of the subject's response to a therapy. In various embodiments, the one or more cells are progenitor cells. Thus, single-cell analysis of the progenitor cells enables a prediction of likely cell lineage of the progenitor cells.

In various embodiments, encapsulating a cell with reagents is accomplished by combining an aqueous phase including the cell and reagents with an immiscible oil phase. In one embodiment, an aqueous phase including the cell and reagents are flowed together with a flowing immiscible oil phase such that water in oil emulsions are formed, where at least one emulsion includes a single cell and the reagents. In various embodiments the immiscible oil phase includes a fluorous oil, a fluorous non-ionic surfactant, or both. In various embodiments, emulsions can have an internal volume of about 0.001 to 1000 picoliters or more and can range from 0.1 to 1000 µm in diameter.

In various embodiments, the aqueous phase including the cell and reagents need not be simultaneously flowing with the immiscible oil phase. For example, the aqueous phase can be flowed to contact a stationary reservoir of the immiscible oil phase, thereby enabling the budding of water in oil emulsions within the stationary oil reservoir.

In various embodiments, combining the aqueous phase and the immiscible oil phase can be performed in a microfluidic device. For example, the aqueous phase can flow through a microchannel of the microfluidic device to contact the immiscible oil phase, which is simultaneously flowing through a separate microchannel or is held in a stationary reservoir of the microfluidic device. The encapsulated cell and reagents within an emulsion can then be flowed through the microfluidic device to undergo cell lysis.

Further example embodiments of adding reagents and cells to emulsions can include merging emulsions that separately contain the cells and reagents or picoinjecting reagents into an emulsion. Further description of example embodiments is described in US Application Publication No. 2015-0232942 A1.

The encapsulated cell in an emulsion is lysed to generate cell lysate. In various embodiments, a cell is lysed by lysing agents that are present in the reagents. For example, the reagents can include a detergent such as NP40 (e.g., Tergitol-type NP-40 or nonyl phenoxypolyethoxylethanol) which lyses the cell membrane. In some embodiments, cell lysis may also, or instead, rely on techniques that do not involve a lysing agent in the reagent. For example, lysis may be achieved by mechanical techniques that may employ various geometric features to effect piercing, shearing, abrading, etc. of cells. Other types of mechanical breakage such as acoustic techniques may also be used. Further, thermal energy can also be used to lyse cells. Any convenient means of effecting cell lysis may be employed in the methods described herein.

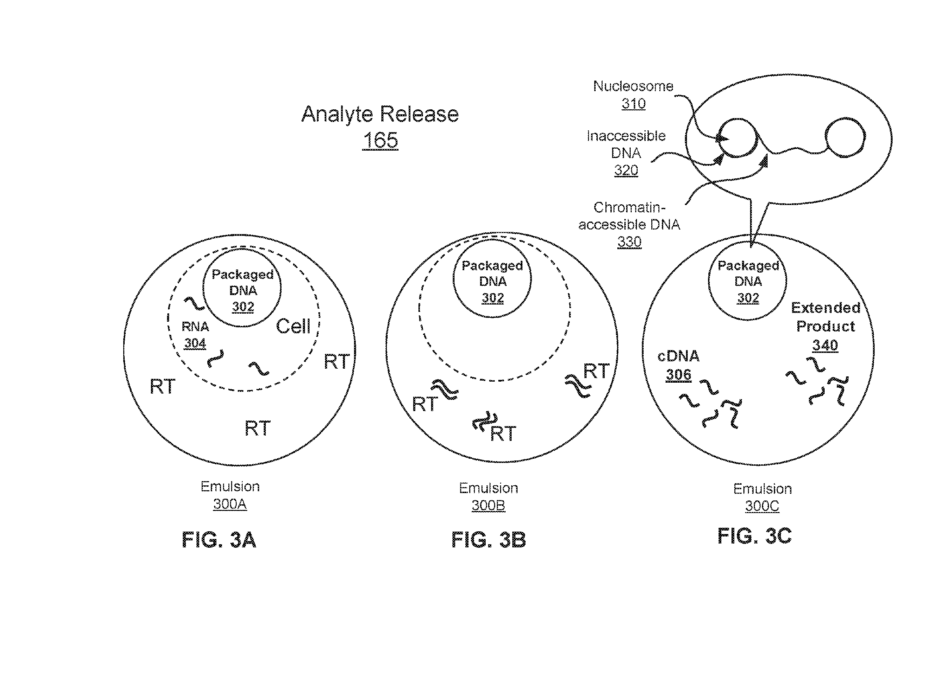

Reference is now made to FIGs. 3A-3C, which depict the processing and releasing of analytes and subsequently processing analytes of a single cell within an emulsion, in accordance with a first embodiment. Specifically, in the embodiment shown in FIGs. 3A-3C, the reagents encapsulated with the cell in the emulsion 300A do not include either proteases (e.g., proteinase K) or transposases (e.g., transposase Tn5). In FIG. 3A, the cell is lysed, as indicated by the dotted line of the cell membrane. In one embodiment, the reagents may include NP40 (e.g., 0.01% or 1.0% NP40) which causes the cell to lyse. The lysed cell includes analytes such as RNA transcripts within the cytoplasm of the cell as well as packaged DNA, which refers to the organization of DNA with histones, thereby forming nucleosomes that are packaged as chromatin. As shown in FIG. 3A, the emulsion 300A further includes reverse transcriptase (abbreviated as "RT").

FIG. 3B depicts the emulsion 300B as reverse transcriptase performs reverse transcription of the RNA transcripts. FIG. 3C depicts the synthesized cDNA strands. Such cDNA strands can be primed using primers included in the reagents, such as reverse primers.

FIG. 3C also depicts the packaged DNA in additional detail. For example, the packaged DNA includes open segments of DNA, herein referred to as chromatin-accessible DNA 330. The chromatin-accessible DNA 330 may reflect a state of the cell, given that gene expression occurs when chromatin-accessible DNA 330 is accessed by the cell transcription machinery (e.g., transcription factors, polymerase, and the like). The packaged DNA further includes nucleosomes 310 including inaccessible DNA 320 that is bound and inaccessible for transcription. In various embodiments, in addition to the packaged DNA 302, emulsion 300C also includes extended products 340 that are generated from the chromatin-accessible DNA 330. For example, segments of the chromatin-accessible DNA 330 can be primed and a complementary DNA strand (e.g., the extended product 340) can be generated. However, in other embodiments, emulsion 300C includes cDNA 306 and packaged DNA 302 but does not include the extended products 340.

The step of cell barcoding 170 in FIG. 1 includes encapsulating a cell lysate 130 with a reaction mixture 140 and a barcode 145. In various embodiments, the reaction mixture 140 includes components, such as primers, for performing a nucleic acid reaction on target nucleic acids (e.g., cDNA or chromatin-accessible DNA).

In various embodiments, a cell lysate is encapsulated with a reaction mixture and a barcode by combining an aqueous phase including the reaction mixture and the barcode with the cell lysate and an immiscible oil phase. In one embodiment, an aqueous phase including the reaction mixture and the barcode are flowed together with a flowing cell lysate and a flowing immiscible oil phase such that water in oil emulsions are formed, where at least one emulsion includes a cell lysate, the reaction mixture, and the barcode. In various embodiments the immiscible oil phase includes a fluorous oil, a fluorous non-ionic surfactant, or both. In various embodiments, emulsions can have an internal volume of about 0.001 to 1000 picoliters or more and can range from 0.1 to 1000 µm in diameter.

In various embodiments, combining the aqueous phase and the immiscible oil phase can be performed in a microfluidic device. For example, the aqueous phase can flow through a microchannel of the microfluidic device to contact the immiscible oil phase, which is simultaneously flowing through a separate microchannel or is held in a stationary reservoir of the microfluidic device. The encapsulated cell lysate, reaction mixture, and barcode within an emulsion can then be flowed through the microfluidic device to perform amplification of target nucleic acids.

Further example embodiments of adding reaction mixture and barcodes to emulsions can include merging emulsions that separately contain the cell lysate and reaction mixture and barcodes or picoinjecting the reaction mixture and/or barcode into an emulsion. Further description of example embodiments of merging emulsions or picoinjecting substances into an emulsion is found in US Application Publication No. 2015-0232942 A1.

Once the reaction mixture and barcode are added to an emulsion, the emulsion may be incubated under conditions that facilitates the nucleic acid amplification reaction. In various embodiments, the emulsion may be incubated on the same microfluidic device as was used to add the reaction mixture and/or barcode, or may be incubated on a separate device. In certain embodiments, incubating the emulsion under conditions that facilitates nucleic acid amplification is performed on the same microfluidic device used to encapsulate the cells and lyse the cells. Incubating the emulsions may take a variety of forms. In certain aspects, the emulsions containing the reaction mix, barcode, and cell lysate may be flowed through a channel that incubates the emulsions under conditions effective for nucleic acid amplification. Flowing the microdroplets through a channel may involve a channel that snakes over various temperature zones maintained at temperatures effective for PCR. Such channels may, for example, cycle over two or more temperature zones, wherein at least one zone is maintained at about 65° C. and at least one zone is maintained at about 95° C. As the drops move through such zones, their temperature cycles, as needed for nucleic acid amplification. The number of zones, and the respective temperature of each zone, may be readily determined by those of skill in the art to achieve the desired nucleic acid amplification.

In various embodiments, following nucleic acid amplification, emulsions containing the amplified nucleic acids are collected. In various embodiments, the emulsions are collected in a well, such as a well of a microfluidic device. In various embodiments, the emulsions are collected in a reservoir or a tube, such as an Eppendorf tube. Once collected, the amplified nucleic acids across the different emulsions are pooled. In one embodiment, the emulsions are broken by providing an external stimuli to pool the amplified nucleic acids. In one embodiment, the emulsions naturally aggregate over time given the density differences between the aqueous phase and immiscible oil phase. Thus, the amplified nucleic acids pool in the aqueous phase.

Following pooling, the amplified nucleic acids can undergo further preparation for sequencing. For example, sequencing adapters can be added to the pooled nucleic acids. Example sequencing adapters are P5 and P7 sequencing adapters. The sequencing adapters enable the subsequent sequencing of the nucleic acids.

### Example Processing of RNA and Chromatin-Accessible DNA

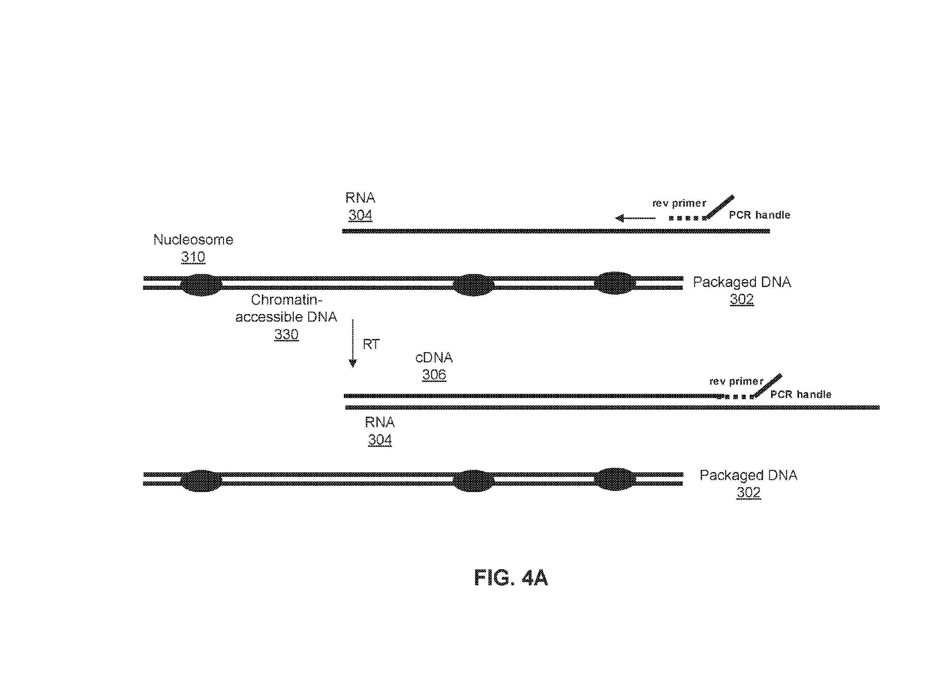

FIG. 4A depicts the processing of RNA and packaged DNA in a first emulsion, in accordance with a first embodiment. Specifically, FIG. 4A depicts, in further detail, the process of analyte release 165 shown in FIG. 1 and FIGs. 3A-3C. Although only a single RNA molecule and a single double-stranded packaged DNA is shown, one skilled in the art would recognize that a single cell can contain significantly more than one RNA molecule and more than one packaged DNA molecule, and therefore, the subsequent description applies across additional RNA molecules and additional packaged DNA molecules.

As described before, the cell is lysed, thereby exposing the cell lysate to the reagents. Here, as shown in the top diagram of FIG. 4A, the cell lysate includes RNA 304 and packaged DNA 302 which comprises nucleosomes 310 and chromatin-accessible DNA 330. The reagents include primers, such as a reverse primer (shown as dotted line) that hybridizes with a segment of the RNA 304. In various embodiments, such a reverse primer is an oligo-dT sequence that hybridizes with a poly-A tail of the messenger RNA transcript. Additionally, the reverse primer includes a PCR handle. The reverse primer primes the RNA 304 molecule. Therefore, as shown in the bottom diagram of FIG. 4A, a cDNA 306 that is complementary to the RNA 304 is generated. In this embodiment, the packaged DNA 302 is not primed and therefore, remains unchanged.

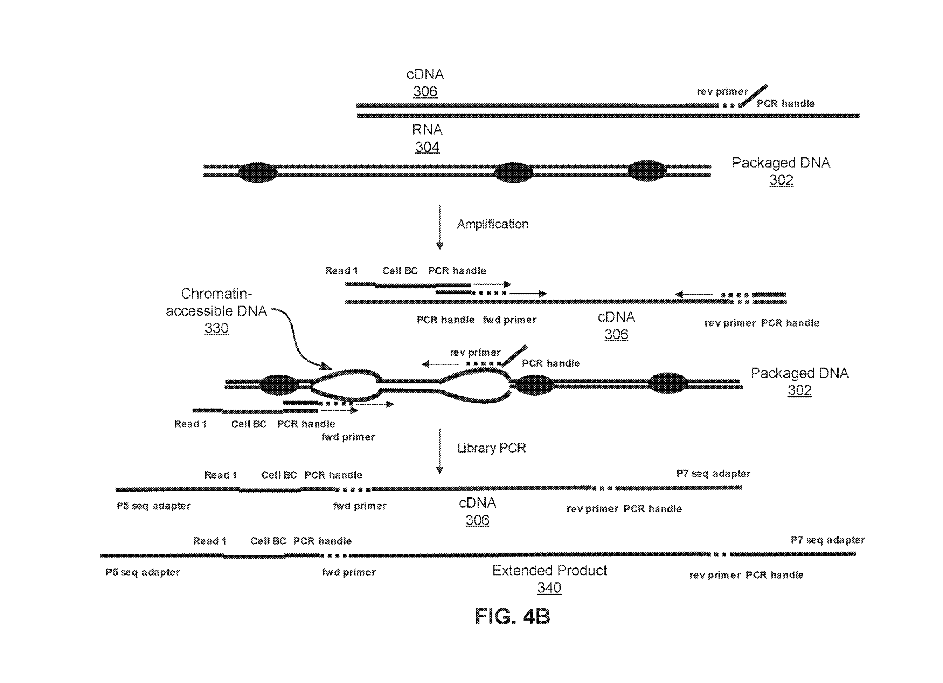

FIG. 4B depicts the amplification and barcoding of nucleic acids derived from RNA and chromatin accessible DNA, in accordance with the first embodiment shown in FIG. 4A. Here, FIG. 4B describes, in further detail, the steps of cell barcoding 170 and target amplification shown in FIG. 1. The top panel of FIG. 4B shows the generated cDNA 306 and packaged DNA 302, as was depicted in the bottom panel of FIG. 4A. The middle panel of FIG. 4B shows the amplification and barcoding process of the cDNA 306 and chromatin accessible DNA 330. Referring first to the cDNA 306, a forward primer and reverse primer pair (forward and reverse primers shown as dotted lines) provided from the reaction mixture can prime the cDNA. The forward primer and reverse primer can be linked to constant regions, such as PCR handles. The PCR handle of the forward primer is complementary to a PCR handle linked to a barcode sequence (annotated as "Cell BC" in FIG. 4B). Thus, forward and reverse synthesis can occur off of the forward and reverse primers as indicated by the horizontal arrows shown in the middle panel.

Referring to the chromatin-accessible DNA 330, segments of the chromatin-accessible DNA 330 are accessible by the reverse primer and forward primer (forward and reverse primers shown as dotted lines) provided from the reaction mixture. In various embodiments, the chromatin-accessible DNA 330 is accessible due to breathing fluctuations of DNA that plays a role for DNA accessibility by regulatory complexes. Further description of the breathing fluctuations of DNA is found in "von Hippel PH, Johnson NP, Marcus AH. Fifty years of DNA "breathing": Reflections on old and new approaches. Biopolymers. 2013;99(12):923-954". The forward primer is linked to a constant region, such as a PCR handle, that is further complementary to a PCR handle linked to a barcode sequence. Thus, forward and reverse synthesis can occur off of the forward and reverse primers as indicated by the horizontal arrows shown in the middle panel.

In particular, the synthesized amplicon from the chromatin-accessible DNA is longer (in comparison to the RNA amplicon) since the forward and reverse primers target different exons. Therefore, the DNA amplicon will contain intronic sequences which, as described below in Example 1, can verify that priming off of chromatin-accessible DNA is occurring.

The bottom panel of FIG. 4B depicts the preparation of the amplified nucleic acids (from the cDNA 306 and from the chromatin-accessible DNA 330). Here, the amplified nucleic acid from cDNA includes sequences of a P5 sequence adapter, a read 1, the barcode ("Cell BC"), the first PCR handle, a forward primer (shown as dotted line), the cDNA, the reverse primer (shown as dotted line), the second PCR handle, and a P7 sequence adapter. The amplified nucleic acid from chromatin-accessible DNA 330 includes sequences of a P5 sequence adapter, a read 1, the barcode ("Cell BC"), the first PCR handle, a forward primer, the extended product 340 (derived from chromatin accessible DNA 330), the reverse primer, the second PCR handle, and a P7 sequence adapter. In various embodiments, a read 2 sequence can be included in each amplified nucleic acid. In one scenario, the read 2 sequence can be included in the second PCR handle linked to the reverse primer sequence. In another scenario, the read 2 sequence can be included in the P7 sequence adapter.

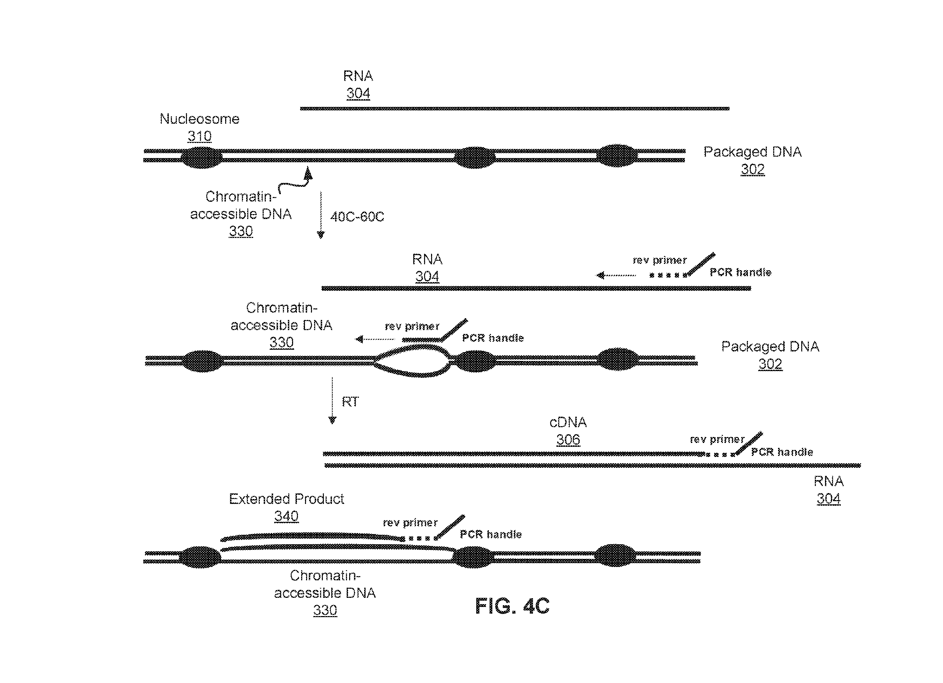

Reference is now made to FIG. 4C, which depicts the processing of RNA and packaged DNA in a first emulsion, in accordance with a second embodiment. Again, FIG. 4C depicts, in further detail, the process of analyte release 165 shown in FIG. 1 and FIGs. 3A-3C. As shown in the top diagram of FIG. 4C, the cell lysate includes RNA 304 and packaged DNA 302 which comprises nucleosomes 310 and chromatin-accessible DNA 330. The emulsion can be exposed to an increased temperature range (e.g., increased relative to physiological temperatures), such as a temperature between 40°C - 60°C. In various embodiments, the emulsion can be exposed to an increased temperature of 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, or 60°C.

The increased temperature exposure can alter the structure of the packaged DNA 302. For example, as shown in the middle panel of FIG. 4C, segments of the chromatin-accessible DNA 330 of the packaged DNA 302 can unwind. In various embodiments, the unwinding of packaged DNA 302 at increased temperatures mimics breathing fluctuations of DNA that plays a role for DNA accessibility by regulatory complexes. Further description of the breathing fluctuations of DNA is found in "von Hippel PH, Johnson NP, Marcus AH. Fifty years of DNA "breathing": Reflections on old and new approaches. Biopolymers. 2013;99(12):923-954". Altogether, the middle panel represents a state of packaged DNA in a cell that is a snapshot of segments of chromatin-accessible DNA that can be accessed by regulatory elements and available for transcription. The middle panel of FIG. 4C further depicts that reverse primers (shown as dotted line) of the added reagents in the emulsion can hybridize with a complementary sequence of the RNA 304 molecule. In various embodiments, such a reverse primer is an oligo-dT sequence that hybridizes with a poly-A tail of the messenger RNA transcript. Additionally, reverse primers can hybridize with a complementary sequence of the chromatin-accessible DNA 330 that has, at least partially, structurally unwound in in view of the increased temperature.

Referring to the bottom panel of FIG. 4C, a complementary cDNA 306 molecule is synthesized off of the RNA 304 molecule. Additionally, an extended product 340 is synthesized off of the chromatin-accessible DNA 330 beginning at the primed region. Thus, the cDNA 306 and the extended product 340 can be further processed in the subsequent steps (e.g., cell barcoding and target amplification) described herein.

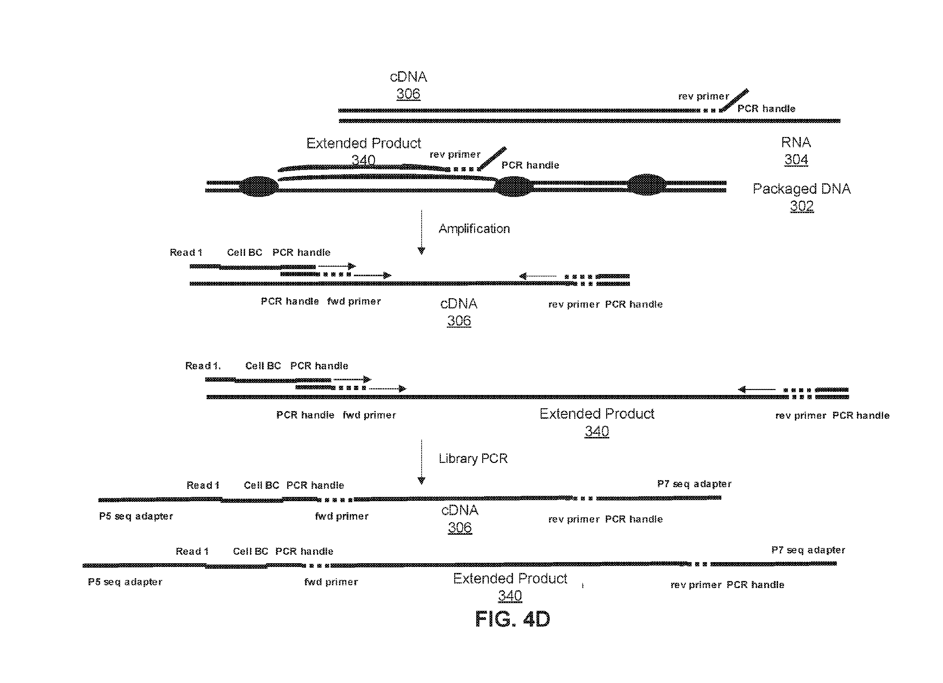

FIG. 4D depicts the amplification and barcoding of nucleic acids derived from RNA and chromatin accessible DNA, in accordance with the second embodiment shown in FIG. 4C. Here, FIG. 4C describes, in further detail, the steps of cell barcoding 170 and target amplification shown in FIG. 1. The top panel of FIG. 4D shows the generated cDNA 306 and extended product 340 off of the packaged DNA 302, as was depicted in the bottom panel of FIG. 4C. The middle panel of FIG. 4D shows the amplification and barcoding process of the cDNA 306 and extended product 340. Generally, the cDNA 306 and extended product 340 are processed in the same manner, given that both are DNA sequences. Referring first to the cDNA 306, a forward primer and reverse primer pair (forward and reverse primers shown as dotted lines) provided from the reaction mixture can prime the cDNA. The forward primer and reverse primer can be linked to constant regions, such as PCR handles. The PCR handle of the forward primer is complementary to a PCR handle linked to a barcode sequence (annotated as "Cell BC" in FIG. 4D). Thus, forward and reverse synthesis can occur off of the forward and reverse primers as indicated by the horizontal arrows shown in the middle panel. Referring to the extended product 340, a forward primer and reverse primer pair provided from the reaction mixture can prime the cDNA. The forward primer and reverse primer can be linked to constant regions, such as PCR handles. The PCR handle of the forward primer is complementary to a PCR handle linked to a barcode sequence (annotated as "Cell BC" in FIG. 4D). Thus, forward and reverse synthesis can occur off of the forward and reverse primers as indicated by the horizontal arrows shown in the middle panel.

The bottom panel of FIG. 4D depicts the preparation of the amplified nucleic acids (from the cDNA 306 and from the chromatin-accessible DNA 330). Here, the amplified nucleic acid from cDNA includes sequences of a P5 sequence adapter, a read 1, the barcode ("Cell BC"), the first PCR handle, a forward primer (shown as dotted line), the cDNA, the reverse primer (shown as dotted line), the second PCR handle, and a P7 sequence adapter. The amplified nucleic acid including the extended product 340 (derived from chromatin-accessible DNA 330) includes sequences of a P5 sequence adapter, a read 1, the barcode ("Cell BC"), the first PCR handle, a forward primer (shown as dotted line), the extended product 340, the reverse primer (shown as dotted line), the second PCR handle, and a P7 sequence adapter. In various embodiments, a read 2 sequence can be included in each amplified nucleic acid. In one scenario, the read 2 sequence can be included in the second PCR handle linked to the reverse primer sequence. In another scenario, the read 2 sequence can be included in the P7 sequence adapter.

### Sequencing and Read Alignment

Amplified nucleic acids are sequenced to obtain sequence reads for generating a sequencing library. Sequence reads can be achieved with commercially available next generation sequencing (NGS) platforms, including platforms that perform any of sequencing by synthesis, sequencing by ligation, pyrosequencing, using reversible terminator chemistry, using phospholinked fluorescent nucleotides, or real-time sequencing. As an example, amplified nucleic acids may be sequenced on an Illumina MiSeq platform.

When pyrosequencing, libraries of NGS fragments are cloned in-situ amplified by capture of one matrix molecule using granules coated with oligonucleotides complementary to adapters. Each granule containing a matrix of the same type is placed in a microbubble of the "water in oil" type and the matrix is cloned amplified using a method called emulsion PCR. After amplification, the emulsion is destroyed and the granules are stacked in separate wells of a titration picoplate acting as a flow cell during sequencing reactions. The ordered multiple administration of each of the four dNTP reagents into the flow cell occurs in the presence of sequencing enzymes and a luminescent reporter, such as luciferase. In the case where a suitable dNTP is added to the 3 ' end of the sequencing primer, the resulting ATP produces a flash of luminescence within the well, which is recorded using a CCD camera. It is possible to achieve a read length of more than or equal to 400 bases, and it is possible to obtain 10⁶ readings of the sequence, resulting in up to 500 million base pairs (megabytes) of the sequence. Additional details for pyrosequencing is described in Voelkerding et al., Clinical Chem., 55: 641-658, 2009; MacLean et al., Nature Rev. Microbiol., 7: 287-296; US patent No. 6,210,891; US patent No. 6,258,568.

On the Solexa / Illumina platform, sequencing data is produced in the form of short readings. In this method, fragments of a library of NGS fragments are captured on the surface of a flow cell that is coated with oligonucleotide anchor molecules. An anchor molecule is used as a PCR primer, but due to the length of the matrix and its proximity to other nearby anchor oligonucleotides, elongation by PCR leads to the formation of a "vault" of the molecule with its hybridization with the neighboring anchor oligonucleotide and the formation of a bridging structure on the surface of the flow cell . These DNA loops are denatured and cleaved. Straight chains are then sequenced using reversibly stained terminators. The nucleotides included in the sequence are determined by detecting fluorescence after inclusion, where each fluorescent and blocking agent is removed prior to the next dNTP addition cycle. Additional details for sequencing using the Illumina platform is found in Voelkerding et al., Clinical Chem., 55: 641-658, 2009; MacLean et al., Nature Rev. Microbiol., 7: 287-296; US patent No. 6,833,246; US patent No. 7,115,400; US patent No. 6,969,488.

Sequencing of nucleic acid molecules using SOLiD technology includes clonal amplification of the library of NGS fragments using emulsion PCR. After that, the granules containing the matrix are immobilized on the derivatized surface of the glass flow cell and annealed with a primer complementary to the adapter oligonucleotide. However, instead of using the indicated primer for 3 'extension, it is used to obtain a 5' phosphate group for ligation for test probes containing two probe-specific bases followed by 6 degenerate bases and one of four fluorescent labels. In the SOLiD system, test probes have 16 possible combinations of two bases at the 3 'end of each probe and one of four fluorescent dyes at the 5' end. The color of the fluorescent dye and, thus, the identity of each probe, corresponds to a certain color space coding scheme. After many cycles of alignment of the probe, ligation of the probe and detection of a fluorescent signal, denaturation followed by a second sequencing cycle using a primer that is shifted by one base compared to the original primer. In this way, the sequence of the matrix can be reconstructed by calculation; matrix bases are checked twice, which leads to increased accuracy. Additional details for sequencing using SOLiD technology is found in Voelkerding et al., Clinical Chem., 55: 641-658, 2009; MacLean et al., Nature Rev. Microbiol., 7: 287-296; US patent No. 5,912,148; US patent No. 6,130,073.

In particular embodiments, HeliScope from Helicos BioSciences is used. Sequencing is achieved by the addition of polymerase and serial additions of fluorescentlylabeled dNTP reagents. Switching on leads to the appearance of a fluorescent signal corresponding to dNTP, and the specified signal is captured by the CCD camera before each dNTP addition cycle. The reading length of the sequence varies from 25-50 nucleotides with a total yield exceeding 1 billion nucleotide pairs per analytical work cycle. Additional details for performing sequencing using HeliScope is found in Voelkerding et al., Clinical Chem., 55: 641-658, 2009; MacLean et al., Nature Rev. Microbiol., 7: 287-296; US Patent No. 7,169,560; US patent No. 7,282,337; US patent No. 7,482,120; US patent No. 7,501,245; US patent No. 6,818,395; US patent No. 6,911,345; US patent No. 7,501,245.

In some embodiments, a Roche sequencing system 454 is used. Sequencing 454 involves two steps. In the first step, DNA is cut into fragments of approximately 300-800 base pairs, and these fragments have blunt ends. Oligonucleotide adapters are then ligated to the ends of the fragments. The adapter serve as primers for amplification and sequencing of fragments. Fragments can be attached to DNA-capture beads, for example, streptavidincoated beads, using, for example, an adapter that contains a 5'-biotin tag. Fragments attached to the granules are amplified by PCR within the droplets of an oil-water emulsion. The result is multiple copies of cloned amplified DNA fragments on each bead. At the second stage, the granules are captured in wells (several picoliters in volume). Pyrosequencing is carried out on each DNA fragment in parallel. Adding one or more nucleotides leads to the generation of a light signal, which is recorded on the CCD camera of the sequencing instrument. The signal intensity is proportional to the number of nucleotides included. Pyrosequencing uses pyrophosphate (PPi), which is released upon the addition of a nucleotide. PPi is converted to ATP using ATP sulfurylase in the presence of adenosine 5 'phosphosulfate. Luciferase uses ATP to convert luciferin to oxyluciferin, and as a result of this reaction, light is generated that is detected and analyzed. Additional details for performing sequencing 454 is found in Margulies et al. (2005) Nature 437: 376-380.

Ion Torrent technology is a DNA sequencing method based on the detection of hydrogen ions that are released during DNA polymerization. The microwell contains a fragment of a library of NGS fragments to be sequenced. Under the microwell layer is the hypersensitive ion sensor ISFET. All layers are contained within a semiconductor CMOS chip, similar to the chip used in the electronics industry. When dNTP is incorporated into a growing complementary chain, a hydrogen ion is released that excites a hypersensitive ion sensor. If homopolymer repeats are present in the sequence of the template, multiple dNTP molecules will be included in one cycle. This results in a corresponding amount of hydrogen atoms being released and in proportion to a higher electrical signal. This technology is different from other sequencing technologies that do not use modified nucleotides or optical devices. Additional details for Ion Torrent Technology is found in Science 327 (5970): 1190 (2010); US Patent Application Publication Nos. 20090026082, 20090127589, 20100301398, 20100197507, 20100188073, and 20100137143.

In various embodiments, sequencing reads obtained from the NGS methods can be filtered by quality and grouped by barcode sequence using any algorithms known in the art, e.g., Python script barcodeCleanup.py . In some embodiments, a given sequencing read may be discarded if more than about 20% of its bases have a quality score (Q-score) less than Q20, indicating a base call accuracy of about 99%. In some embodiments, a given sequencing read may be discarded if more than about 5%, about 10%, about 15%, about 20%, about 25%, about 30% have a Q-score less than Q10, Q20, Q30, Q40, Q50, Q60, or more, indicating a base call accuracy of about 90%, about 99%, about 99.9%, about 99.99%, about 99.999%, about 99.9999%, or more, respectively.

In some embodiments, all sequencing reads associated with a barcode containing less than 50 reads may be discarded to ensure that all barcode groups, representing single cells, contain a sufficient number of high-quality reads. In some embodiments, all sequencing reads associated with a barcode containing less than 30, less than 40, less than 50, less than 60, less than 70, less than 80, less than 90, less than 100 or more may be discarded to ensure the quality of the barcode groups representing single cells.

Sequence reads with common barcode sequences (e.g., meaning that sequence reads originated from the same cell) may be aligned to a reference genome using known methods in the art to determine alignment position information. The alignment position information may indicate a beginning position and an end position of a region in the reference genome that corresponds to a beginning nucleotide base and end nucleotide base of a given sequence read. A region in the reference genome may be associated with a target gene or a segment of a gene. Example aligner algorithms include BWA, Bowtie, Spliced Transcripts Alignment to a Reference (STAR), Tophat, or HISAT2. Further details for aligning sequence reads to reference sequences is described in US Application Publication No. 2020-0051663 A1. In various embodiments, an output file having SAM (sequence alignment map) format or BAM (binary alignment map) format may be generated and output for subsequent analysis, such as for determining cell trajectory.

### Determining Cell Trajectory

Sequencing reads of nucleic acids derived from RNA transcripts and chromatin-accessible DNA of a single cell are analyzed to determine a cell trajectory of the single cell. Generally, the cell trajectory refers to a change of a cell from a first state to a second state as represented by the cell's chromatin structure. Thus, the cell trajectory is a reflection of a chromatin organization profile of a cell. Sequencing reads obtained through RNA-seq provide a snapshot of a past state of the cell. For example, the presence of RNA transcripts can reveal details of past chromatin organization (e.g., certain genes corresponding to the RNA transcripts are expressed and are therefore accessible in the chromatin). Sequencing reads obtained through DNA-seq provide a snapshot of a future state of the cell. For example, the DNA-seq results reveal details of the current chromatin organization (e.g., certain genes corresponding to chromatin-accessible regions can be available for transcription and expression). Altogether, the RNA-seq and DNA-seq sequence reads reveal a chromatin structure profile.

To determine a cell trajectory, aligned sequence reads derived from RNA transcripts are compared to aligned sequence reads derived from chromatin-accessible DNA. More specifically, read counts of sequence reads derived from RNA transcripts are compared to read counts of aligned sequence reads derived from chromatin-accessible DNA. In various embodiments, the comparison is conducted on an individual gene basis.

For example, for a gene with a known range of positions in the reference genome, there may be "X" sequence reads obtained through RNA-seq and "Y" sequence reads obtained through DNA-seq. In one embodiment, the "X" sequence reads obtained through RNA-seq indicates expression of the gene and the "Y" sequence reads obtained through DNA-seq indicates that the gene corresponds to a chromatin accessible region of the DNA. This reveals a commonality between the prior state of the cell (as revealed through RNA-seq) and the future state of the cell (as revealed through DNA-seq) given that the gene is accessible in the packaged DNA in both the prior state and the future state.

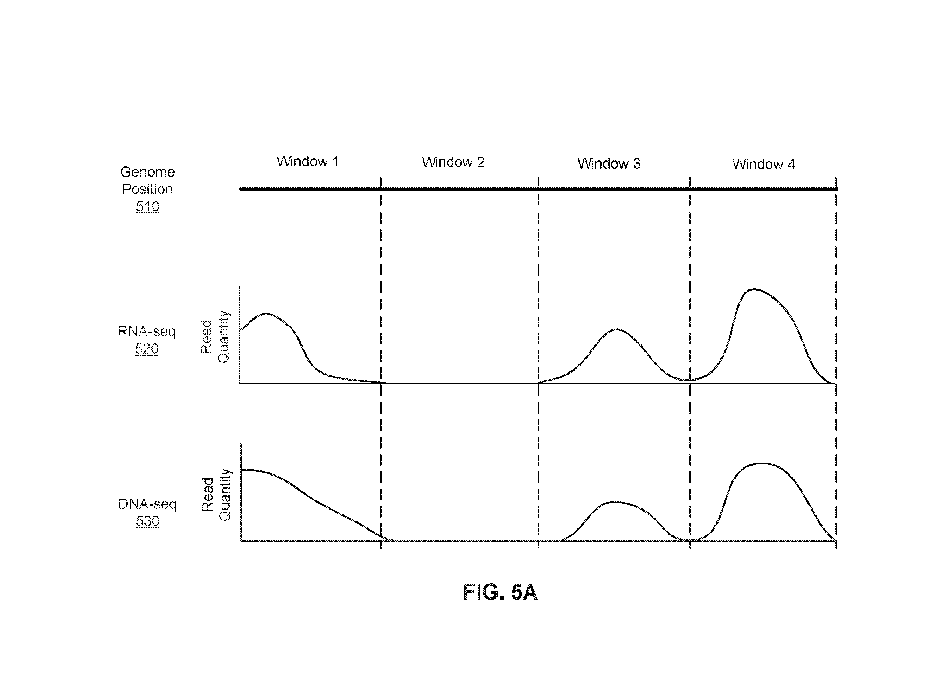

Reference is made to FIG. 5A which depicts sequencing reads obtained through single-cell RNA-seq and DNA-seq for determining a cell trajectory, in accordance with a first embodiment. FIG. 5A depicts four windows of a reference genome. Each window includes a range of positions along the genome. In various embodiments, each window can refer to a range of positions for a known gene. The second and third graphs show read quantity for RNA-seq 520 and DNA-seq 530 respectively across the genome positions in each of the four windows. Here, both RNA-seq and DNA-seq results in sequence reads in window 1, 3, and 4 with no sequence reads in window 2. For example, if each of the four windows refers to positions of a gene, the RNA-seq 520 and DNA-seq 530 reads demonstrate commonality between the prior state and future state of the four genes. Namely, the chromatin structure profile of the four genes is unchanged. This is informative for identifying a cell trajectory in which the chromatin structure profile for the four genes remains unchanged.

In another embodiment, the "X" sequence reads obtained through RNA-seq and the "Y" sequence reads reveals a transition from the prior state of the cell and the future state of the cell. As one example, the "X" sequence reads obtained through RNA-seq indicates that the gene was not expressed. In such a scenario, X = 0 sequence reads or nearly zero sequence reads. However, the "Y" sequence reads obtained through DNA-seq indicates that the gene is accessible in the packaged DNA and available for transcription. This may indicate that the cell is transitioning from a prior state of non-expression of the gene to a future state in which the gene may be expressed. As another example, the "X" sequence reads obtained through RNA-seq indicates that the gene was expressed. However, the "Y" sequence reads obtained through DNA-seq indicates that the gene is not accessible in the packaged DNA and is unavailable for transcription. In such a scenario, Y = 0 sequence reads or nearly zero sequence reads. This may indicate that the cell is transitioning from a prior state of expression of the gene to a future state in which the gene is not expressed.

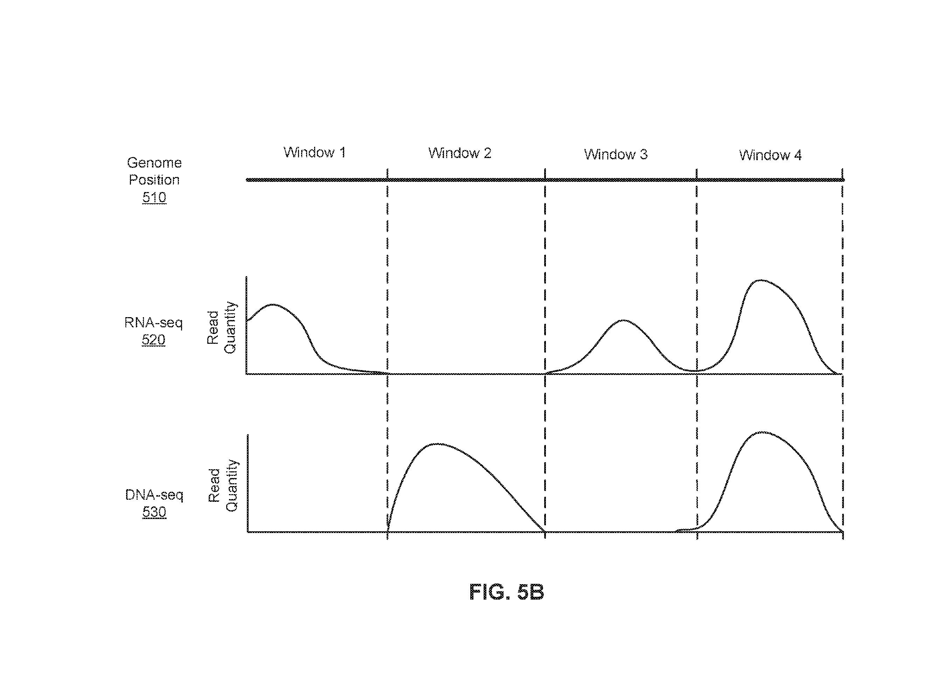

FIG. 5B depicts sequencing reads obtained through single-cell RNA-seq and DNA-seq for determining a cell trajectory, in accordance with a second embodiment. Again, FIG. 5B depicts four windows of a reference genome. Each window includes a range of positions along the genome. In various embodiments, each window can refer to a range of positions for a known gene. The second and third graphs show read quantity for RNA-seq 520 and DNA-seq 530 respectively across the genome positions in each of the four windows.

Here, the RNA-seq and DNA-seq result in differing read quantities in windows 1, 2, and 3, whereas read quantities in window 4 are generally in agreement. Again, if each of the four windows refers to positions of a gene, the RNA-seq 520 and DNA-seq 530 reads demonstrate a transition in the chromatin profile for three of the genes (e.g., windows 1, 2, and 3) whereas the chromatin profile for the fourth gene (e.g., window 4) is unchanged. Specifically, windows 1 and 3 indicate that the corresponding genes may have transitioned from an accessible state to a non-accessible state. Window 2 indicates that the corresponding gene may have transitioned from a non-accessible state to an accessible state. This is informative for identifying a cell trajectory in which the chromatin structure profile undergoes a transition (e.g., accessible to non-accessible or non-accessible to accessible) for three of the genes and unchanged for a fourth gene.

Although the preceding example description refers to a single gene or to a limited number of genes (e.g., four genes shown in FIG. 5A and 5B), the analysis of sequence reads obtained through RNA-seq and DNA-seq can be applied to tens, hundreds, thousands, or tens of thousands of genes across the genome. As such, for each cell, the unchanged or changed chromatin structure profile across the cell's genome can be determined. Unchanging or changing chromatin structure profile is informative for determining the cell trajectory, such as any of a cell lineage, a cell fate, a cell function in a future state of the cell, a diseased future state of the cell, a future cellular response to an external stimulus (e.g., a treatment). Further description of using a chromatin profile to predict a cell lineage is found in Ma et al., Chromatin potential identified by shared single cell profiling of RNA and chromatin, bioRxiv, June 18, 2020, doi: https://doi.org/10.1101/2020.06.17.156943.

### Barcodes and Barcoded Beads

Embodiments of the invention involve providing one or more barcode sequences for labeling analytes of a single cell during step 170 shown in FIG. 1. The one or more barcode sequences are encapsulated in an emulsion with a cell lysate derived from a single cell. As such, the one or more barcodes label analytes of the cell, thereby enabling the subsequent determination that sequence reads derived from the analytes originated from the cell.

In various embodiments, a plurality of barcodes are added to an emulsion with a cell lysate. In various embodiments, the plurality of barcodes added to an emulsion includes at least 10², at least 10³, at least 10⁴, at least 10⁵, at least 10⁵, at least 10⁶, at least 10⁷, or at least 10⁸ barcodes. In various embodiments, the plurality of barcodes added to an emulsion have the same barcode sequence. In various embodiments, the plurality of barcodes added to an emulsion comprise a 'unique identification sequence' (UMI). A UMI is a nucleic acid having a sequence which can be used to identify and/or distinguish one or more first molecules to which the UMI is conjugated from one or more second molecules. UMIs are typically short, e.g., about 5 to 20 bases in length, and may be conjugated to one or more target molecules of interest or amplification products thereof. UMIs may be single or double stranded. In some embodiments, both a barcode sequence and a UMI are incorporated into a barcode. Generally, a UMI is used to distinguish between molecules of a similar type within a population or group, whereas a barcode sequence is used to distinguish between populations or groups of molecules that are derived from different cells. In some embodiments, where both a UMI and a barcode sequence are utilized, the UMI is shorter in sequence length than the barcode sequence. The use of barcodes is further described in US Patent Application Publication No.2018-0216160.

In some embodiments, the barcodes are single-stranded barcodes. Single-stranded barcodes can be generated using a number of techniques. For example, they can be generated by obtaining a plurality of DNA barcode molecules in which the sequences of the different molecules are at least partially different. These molecules can then be amplified so as to produce single stranded copies using, for instance, asymmetric PCR. Alternatively, the barcode molecules can be circularized and then subjected to rolling circle amplification. This will yield a product molecule in which the original DNA barcoded is concatenated numerous times as a single long molecule.

In some embodiments, circular barcode DNA containing a barcode sequence flanked by any number of constant sequences can be obtained by circularizing linear DNA. Primers that anneal to any constant sequence can initiate rolling circle amplification by the use of a strand displacing polymerase (such as Phi29 polymerase), generating long linear concatemers of barcode DNA.

In various embodiments, barcodes can be linked to a primer sequence that enables the barcode to label a target nucleic acid. In one embodiment, the barcode is linked to a forward primer sequence. In various embodiments, the forward primer sequence is a gene specific primer that hybridizes with a forward target of a nucleic acid. In various embodiments, the forward primer sequence is a constant region, such as a PCR handle, that hybridizes with a complementary sequence attached to a gene specific primer. The complementary sequence attached to a gene specific primer can be provided in the reaction mixture (e.g., reaction mixture 140 in FIG. 1). Including a constant forward primer sequence on barcodes may be preferable as the barcodes can have the same forward primer and need not be individually designed to be linked to gene specific forward primers.

In various embodiments, barcodes can releasably attached to a support structure, such as a bead. Therefore, a single bead with multiple copies of barcodes can be partitioned into an emulsion with a cell lysate, thereby enabling labeling of analytes of the cell lysate with the barcodes of the bead. Example beads include solid beads (e.g., silica beads), polymeric beads, or hydrogel beads (e.g., polyacrylamide, agarose, or alginate beads). Beads can be synthesized using a variety of techniques. For example, using a mix-split technique, beads with many copies of the same, random barcode sequence can be synthesized. This can be accomplished by, for example, creating a plurality of beads including sites on which DNA can be synthesized. The beads can be divided into four collections and each mixed with a buffer that will add a base to it, such as an A, T, G, or C. By dividing the population into four subpopulations, each subpopulation can have one of the bases added to its surface. This reaction can be accomplished in such a way that only a single base is added and no further bases are added. The beads from all four subpopulations can be combined and mixed together, and divided into four populations a second time. In this division step, the beads from the previous four populations may be mixed together randomly. They can then be added to the four different solutions, adding another, random base on the surface of each bead. This process can be repeated to generate sequences on the surface of the bead of a length approximately equal to the number of times that the population is split and mixed. If this was done 10 times, for example, the result would be a population of beads in which each bead has many copies of the same random 10-base sequence synthesized on its surface. The sequence on each bead would be determined by the particular sequence of reactors it ended up in through each mix-split cycle. Additional details of example beads and their synthesis is described in International Application Publication No. WO-A-2016/126871.

### Reagents

Embodiments described herein include the encapsulation of a cell with reagents within an emulsion. Generally, the reagents interact with the encapsulated cell under conditions in which the cell is lysed, thereby releasing target analytes of the cell. The reagents can further interact with target analytes to prepare for subsequent barcoding and/or amplification. In various embodiments, the reagents include ddNTPs, inhibitors such as ribonuclease inhibitor, primers (e.g., reverse primers such as oligodT or gene specific reverse primers), and stabilization agents such as dithothreitol (DTT).

In various embodiments, the reagents include one or more lysing agents that cause the cell to lyse. Examples of lysing agents include detergents such as Triton X-100, NP-40, as well as cytotoxins. Examples of NP-40 include Thermo Scientific NP-40 Surfact-Amps Detergent solution and Sigma Aldrich NP-40 (TERGITOL Type NP-40). In some embodiments, the reagents include NP40 detergent which is sufficient to disrupt the cell membrane and cause cell lysis, but does not disrupt chromatin packaged DNA. In various embodiments, the reagents include 0.01%, 0.05%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, or 5.0% NP40 (v/v). In various embodiments, the reagents include at least at least 0.01%, at least 0.05%, 0.1%, at least 0.5%, at least 1%, at least 2%, at least 3%, at least 4%, or at least 5% NP40 (v/v).

In various embodiments, the reagents further include proteases that assist in the lysing of the cell and/or accessing of genomic DNA. In various embodiments, proteases in the reagents can include any of proteinase K, pepsin, protease-subtilisin Carlsberg, protease type X-bacillus thermoproteolyticus, or protease type XIII-aspergillus Saitoi. In various embodiments, the quantity of protease in the reagents is less than the quantity of protease used in single-cell workflow protocols. In various embodiments, the quantity of protease in the reagents is less than 0.01%, less than 0.05%, less than 0.1%, less than 0.2%, less than 0.3%, less than 0.4%, less than 0.5%, less than 0.6%, less than 0.7%, less than 0.8%, less than 0.9%, less than 1%, less than 2%, less than 3%, less than 4%, less than 5%, less than 10%, less than 15%, less than 20%, less than 25%, less than 30%, less than 40%, or less than 50% of the amounts present in conventional single-cell workflow protocols. For example, single-cell workflow protocols have used 1 mg/mL proteinase K (see Pellegrino, Maurizio et al. "High-throughput single-cell DNA sequencing of acute myeloid leukemia tumors with droplet microfluidics." Genome research vol. 28,9 (2018): 1345-1352). Thus, in various embodiments, the reagents includes less than 0.0001 mg/mL, less than 0.0005 mg/mL, less than 0.0010 mg/mL, less than 0.0020 mg/mL, less than 0.0030 mg/mL, less than 0.0040 mg/mL, less than 0.0050 mg/mL, less than 0.0060 mg/mL, less than 0.0070 mg/mL, less than 0.0080 mg/mL, less than 0.0090 mg/mL, less than 0.01 mg/mL, less than 0.02 mg/mL, less than 0.03 mg/mL, less than 0.04 mg/mL, less than 0.05 mg/mL, less than 0.10 mg/mL, less than 0.15 mg/mL, less than 0.20 mg/mL, less than 0.30 mg/mL, less than 0.40 mg/mL, or less than 0.50 mg/mL.

In various embodiments, the reagents further include agents that interact with target analytes that are released from a single cell. One example of such an agent includes reverse transcriptase which reverse transcribes messenger RNA transcripts released from the cell to generate corresponding cDNA.

In some embodiments, agents include a transposase Tn5 (or mutated transposase Tn5) which interacts with packaged DNA to generate segments of chromatin accessible DNA that are not bound by chromatin and/or nucleosomes. In various embodiments, the quantity of transposase Tn5 in the reagents is less than the quantity of transposase Tn5 used in conventional ATAC-seq protocols. Examples of transposase Tn5 that are used in conventional ATAC-seq protocols include Illumina Tagment DNA Enzyme (Illumina Catalog Numbers 20034197 or 20034198) and Nextera Tn5 Transposase, Illumina Cat #FC-121-1030. In various embodiments, the quantity of transposase Tn5 in the reagents is less than 0.01%, less than 0.05%, less than 0.1%, less than 0.2%, less than 0.3%, less than 0.4%, less than 0.5%, less than 0.6%, less than 0.7%, less than 0.8%, less than 0.9%, less than 1%, less than 2%, less than 3%, less than 4%, less than 5%, less than 10%, less than 15%, less than 20%, less than 25%, less than 30%, less than 40%, or less than 50% of the amounts present in conventional ATAC-seq protocols. For example, conventional ATAC-seq protocols use 5% transposase Tn5 (v/v) (see Buenrostro, J. et al., "Single-cell chromatin accessibility reveals principles of regulatory variation." Nature, 523(7561): 486-490. July 23, 2015, Buenrostro, J. et al. "ATAC-seq: A Method for Assaying Chromatin Accessibility Genome-Wide." Current protocols in molecular biology vol. 109 21.29.1-21.29.9. 5 Jan. 2015, and Shashikant, Tanvi, and Charles A Ettensohn. "Genome-wide analysis of chromatin accessibility using ATAC-seq." Methods in cell biology vol. 151 (2019): 219-235). Thus, in various embodiments, the reagents includes less than 0.0005%, less than 0.0025%, less than 0.005%, less than 0.01%, less than 0.015%, less than 0.02%, less than 0.025%, less than 0.030%, less than 0.035%, less than 0.040%, less than 0.045%, less than 0.050%, less than 0.1%, less than 0.15%, less than 0.2%, less than 0.25%, less than 0.5%, less than 0.75%, less than 1.0%, less than 1.25%, less than 1.5%, less than 1.75%, less than 2.0%, or less than 2.5% transposase Tn5 (v/v).

Embodiments herein describe performing DNA-sequencing on DNA from single cells. In preferred embodiments, DNA-sequencing is performed on chromatin-accessible DNA (e.g., DNA that can be accessed when packaged with chromatin). In particular embodiments consistent with performing DNA-sequencing on chromatin-accessible DNA, the reagents that are co-encapsulated with a cell do not include a protease and more specifically, do not include a proteinase K (or mutated proteinase K). Furthermore, the reagents do not include a transposase, such as transposase Tn5, or a mutated transposase, such as mutated transposase Tn5.

In particular embodiments, reagents include NP40 and do not include proteinase K or transposase. NP40 is sufficient in lysing the cells without disrupting packaged DNA, and subsequently, priming of chromatin accessible DNA within packaged DNA can be performed which enables DNA-sequencing that interrogates chromatin-accessible DNA. Using reagents that include NP40 and do not include proteinase K or transposase is preferable because the lack of proteinase K and transposase simplifies the single-cell workflow process. Additionally, the lack of proteinase K and transposase results in fewer consumables being used in the workflow process, thereby resulting in lower costs when performing single-cell analysis of a large numbers of cells.

### Reaction Mixture

As described herein, a reaction mixture is provided into an emulsion with a cell lysate (e.g., see cell barcoding step 170 in FIG. 1). Generally, the reaction mixture includes reactants sufficient for performing a reaction, such as nucleic acid amplification, on analytes of the cell lysate.

In various embodiments, the reaction mixture includes primers that are capable of acting as a point of initiation of synthesis along a complementary strand when placed under conditions in which synthesis of a primer extension product which is complementary to a nucleic acid strand is catalyzed. In various embodiments, the reaction mixture includes the four different deoxyribonucleoside triphosphates (adenosine, guanine, cytosine, and thymine). In various embodiments, the reaction mixture includes enzymes for nucleic acid amplification. Examples of enzymes for nucleic acid amplification include DNA polymerase, thermostable polymerases for thermal cycled amplification, or polymerases for multiple-displacement amplification for isothermal amplification. Other, less common forms of amplification may also be applied, such as amplification using DNA- dependent RNA polymerases to create multiple copies of RNA from the original DNA target which themselves can be converted back into DNA, resulting in, in essence, amplification of the target. Living organisms can also be used to amplify the target by, for example, transforming the targets into the organism which can then be allowed or induced to copy the targets with or without replication of the organisms.

In various embodiments, the contents of the reaction mixture are in a suitable buffer ("buffer" includes substituents which are cofactors, or which affect pH, ionic strength, etc.), and at a suitable temperature.

The extent of nucleic amplification can be controlled by modulating the concentration of the reactants in the reaction mixture. In some instances, this is useful for fine tuning of the reactions in which the amplified products are used.

### Primers

Embodiments of the invention described herein use primers to conduct the single-cell analysis. For example, primers are implemented during the workflow process shown in FIG. 1. Primers can be used to prime (e.g., hybridize) with specific sequences of nucleic acids of interest, such that the nucleic acids of interest can be barcoded and/or amplified. Additionally, primers enable the identification of target regions following sequencing. As described hereafter, primers can be provided in the workflow process shown in FIG. 1 in various steps. Referring again to FIG. 1, in various embodiments, primers can be included in the reagents 120 that are encapsulated with the cell 110. Such primers in the reagents 120 can include RNA primers for priming RNA and/or DNA primers for priming chromatin-accessible DNA in packaged DNA. In various embodiments, primers can be included in the reaction mixture 140 that is encapsulated with the cell lysate 130. Such primers in the reaction mixture 140 can include cDNA primers for priming cDNA that have been reverse transcribed from RNA and/or DNA primers for priming chromatin-accessible DNA in packaged DNA and/or products that have been generated from chromatin-accessible DNA. In various embodiments, primers can be included in or linked with a barcode 145 that is encapsulated with the cell lysate 130. Further description and examples of primers that are used in a single-cell analysis workflow process is described in US Application Publication No. 2020-0232011 A1.

In various embodiments, the number of primers in any of the reagents, the reaction mixture, or with barcodes may range from about 1 to about 500 or more, e.g., about 2 to 100 primers, about 2 to 10 primers, about 10 to 20 primers, about 20 to 30 primers, about 30 to 40 primers, about 40 to 50 primers, about 50 to 60 primers, about 60 to 70 primers, about 70 to 80 primers, about 80 to 90 primers, about 90 to 100 primers, about 100 to 150 primers, about 150 to 200 primers, about 200 to 250 primers, about 250 to 300 primers, about 300 to 350 primers, about 350 to 400 primers, about 400 to 450 primers, about 450 to 500 primers, or about 500 primers or more.

For targeted DNA sequencing and targeted RNA sequencing, primers in the reagents (e.g., reagents 120 in FIG. 1) may include reverse primers that are complementary to a reverse target on a nucleic acid of interest (e.g., DNA or RNA). In various embodiments, primers in the reagents may be gene-specific primers that target a reverse target of a gene of interest. In various embodiments, primers in the reaction mixture (e.g., reaction mixture 140 in FIG. 1) may include forward primers that are complementary to a forward target on a nucleic acid of interest (e.g., DNA). In various embodiments, primers in the reaction mixture may be gene-specific primers that target a forward target of a gene of interest. In various embodiments, primers of the reagents and primers of the reaction mixture form primer sets (e.g., forward primer and reverse primer) for a region of interest on a nucleic acid.

The number of forward or reverse primers for genes of interest that are added may be from about one to 500, e.g., about 1 to 10 primers, about 10 to 20 primers, about 20 to 30 primers, about 30 to 40 primers, about 40 to 50 primers, about 50 to 60 primers, about 60 to 70 primers, about 70 to 80 primers, about 80 to 90 primers, about 90 to 100 primers, about 100 to 150 primers, about 150 to 200 primers, about 200 to 250 primers, about 250 to 300 primers, about 300 to 350 primers, about 350 to 400 primers, about 400 to 450 primers, about 450 to 500 primers, or about 500 primers or more. In various embodiments, genes of interest for either DNA-sequencing or RNA-sequencing include, but are not limited to: CCND3, CD44, CCND1, CD33, CDK6, CDK4, CDKN1B, CREB3L4, CDKN1A, CREBBP, CREB3L1, CREB5, CREB1, ELK1, FOS, FHL1, FASLG, GNG12, GSK3B, BAD, FOXO4, FOXO1, HIF1A, HSPB1, IKBKG, IRF9, BCL2, BCL2L11, MAP2K1 MAPK1, BCL2L1, MYB, NF1, NFKB1, MYC, PIK3CB, PIM1, PIAS1, PRKCB, PTEN, HSPA1A, HSPA2, IL2RB, IL2RA, SIRT1, NCL, RHOA, MCM4, NASP, SOS1, TCL1B, SOCS3, SOCS2, STAT4, STAT6, SRF, TP53, CASP9, CASP3, CASP8, UBB, MPRL16, MRPL21, FAM32A, ABCB7, PCBP1. EPS15, NRAS, RPS27A, AFF3, PAX3, CMTM6, RHOA, PIK3CA, MAP3K13, NSD1, PTPRK, CARD11, EGFR, EZH2, WRN, JAK2, GATA3, DKK1, POLA2, CCND1, ATM, ARHGEF12, KRAS, COL2A1, KMT2D, CLIP1, FLT3, BRCA2, BUB1B, PALB2, FANCA, NCOR1, ERBB2, KAT2A, RAB5C, METTL23, SRSF2, MFSD11, DNM2, CIC, BCR, MYH9, EP300, and SSX1.

For whole transcriptome RNA sequencing, in various embodiments, the primers of the reagents (e.g., reagents 120 in FIG. 1) can include a constant reverse primer and a random forward primer. The constant reverse primer may include a universal primer region and a reverse constant region, such as a PCR handle. For example, the universal primer region can be an oligo dT sequence that hybridizes with the poly A tail of messenger RNA transcripts. This priming enables reverse transcription of the mRNA transcript. The random forward primer may have a random primer sequence that hybridizes with a sequence of reverse transcribed cDNA, thereby enabling priming off of the cDNA. In various embodiments, the primers of the reaction mixture (e.g., reaction mixture 140 in FIG. 1) may be constant forward primers and constant reverse primers. The constant forward primers can hybridize with the random forward primer that enables priming off the cDNA. The constant reverse primers can hybridize with a sequence of the reverse constant region, such as a PCR handle, that previously enabled reverse transcription of the mRNA transcript.

In various embodiments, instead of the primers being included in the reaction mixture (e.g., reaction mixture 140 in FIG. 1) such primers can be included or linked to a barcode (e.g., barcode 145 in FIG. 1). In particular embodiments, the primers are linked to an end of the barcode and therefore, are available to hybridize with target sequences of nucleic acids in the cell lysate.

In various embodiments, primers of the reaction mixture, primers of the reagents, or primers of barcodes may be added to an emulsion in one step, or in more than one step. For instance, the primers may be added in two or more steps, three or more steps, four or more steps, or five or more steps. Regardless of whether the primers are added in one step or in more than one step, they may be added after the addition of a lysing agent, prior to the addition of a lysing agent, or concomitantly with the addition of a lysing agent. When added before or after the addition of a lysing agent, the primers of the reaction mixture may be added in a separate step from the addition of a lysing agent (e.g., as exemplified in the two step workflow process shown in FIG. 1).

A primer set for the amplification of a target nucleic acid typically includes a forward primer and a reverse primer that are complementary to a target nucleic acid or the complement thereof. In some embodiments, amplification can be performed using multiple target-specific primer pairs in a single amplification reaction, wherein each primer pair includes a forward target-specific primer and a reverse target-specific primer, where each includes at least one sequence that substantially complementary or substantially identical to a corresponding target sequence in the sample, and each primer pair having a different corresponding target sequence. Accordingly, certain methods herein are used to detect or identify multiple target sequences from a single cell sample.

### Example System and/or Computer Embodiments

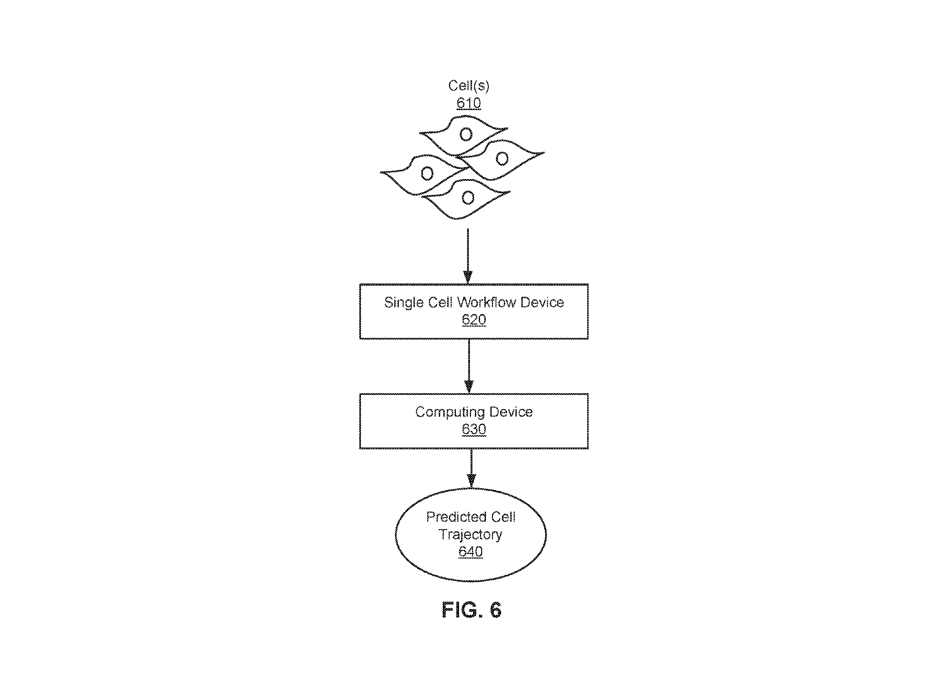

FIG. 6 depicts an overall system environment including a single cell workflow device 620 and a computational device 630 for conducting single-cell analysis on cell(s) to generate a predicted cell trajectory 640, in accordance with the embodiments described in FIGs. 1-5. In various embodiments, the single cell workflow device 620 is configured to perform the steps of cell encapsulation 160, analytes release 165, cell barcoding 170, target amplification 175, nucleic acid pooling 205, and sequencing 210. In various embodiments, the computing device 630 is configured to perform the *in silico* steps of read alignment 215 and determining cell trajectory 220.

In various embodiments, a single cell workflow device 620 includes at least a microfluidic device that is configured to encapsulate cells with reagents, encapsulate cell lysates with reaction mixtures, and perform nucleic acid amplification reactions. For example, the microfluidic device can include one or more fluidic channels that are fluidically connected. Therefore, the combining of an aqueous fluid through a first channel and a carrier fluid through a second channel results in the generation of emulsion droplets. In various embodiments, the fluidic channels of the microfluidic device may have at least one crosssectional dimension on the order of a millimeter or smaller (e.g., less than or equal to about 1 millimeter). Additional details of microchannel design and dimensions is described in International Patent Application Publication No. WO-A-2016/126871 and US Patent Application Publication No. 2015-0232942 A1. An example of a microfluidic device is the Tapestri^{™} Platform.

In various embodiments, the single cell workflow device 620 may also include one or more of: (a) a temperature control module for controlling the temperature of one or more portions of the subject devices and/or droplets therein and which is operably connected to the microfluidic device(s), (b) a detection means, i.e., a detector, e.g., an optical imager, operably connected to the microfluidic device(s), (c) an incubator, e.g., a cell incubator, operably connected to the microfluidic device(s), and (d) a sequencer operably connected to the microfluidic device(s). The one or more temperature and/or pressure control modules provide control over the temperature and/or pressure of a carrier fluid in one or more flow channels of a device. As an example, a temperature control module may be one or more thermal cycler that regulates the temperature for performing nucleic acid amplification. The one or more detection means i.e., a detector, e.g., an optical imager, are configured for detecting the presence of one or more droplets, or one or more characteristics thereof, including their composition. In some embodiments, detection means are configured to recognize one or more components of one or more droplets, in one or more flow channel. The sequencer is a hardware device configured to perform sequencing, such as next generation sequencing. Examples of sequencers include Illumina sequencers (e.g., MiniSeq^{™}, MiSeq^{™}, NextSeq^{™} 550 Series, or NextSeq^{™} 2000), Roche sequencing system 454, and Thermo Fisher Scientific sequencers (e.g., Ion GeneStudio S5 system, Ion Torrent Genexus System).

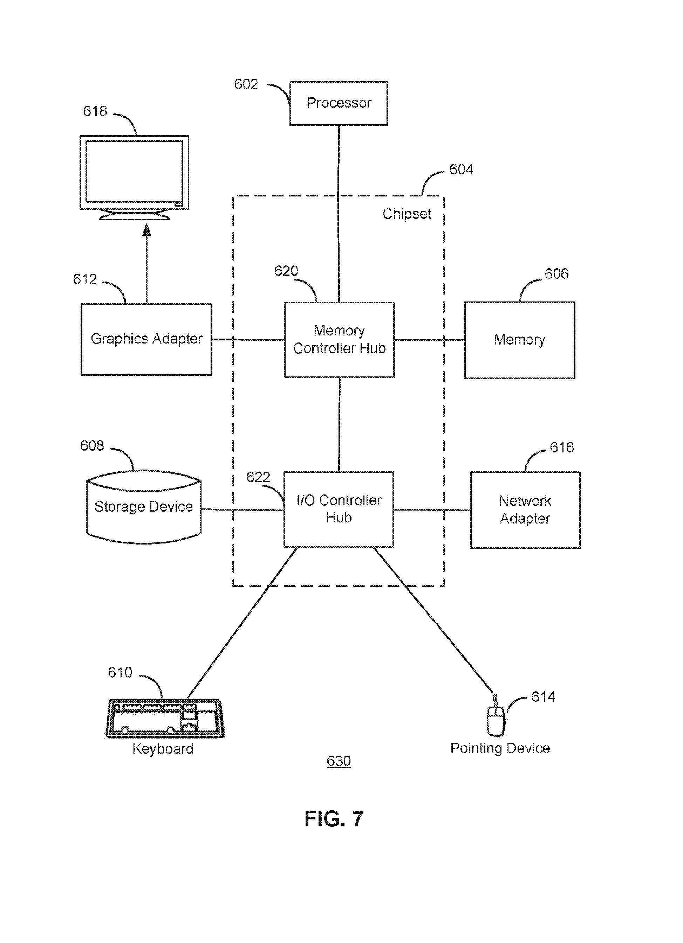

FIG. 7 depicts an example computing device for implementing system and methods described in reference to FIGs. 1-6. For example, the example computing device 630 is configured to perform the *in silico* steps of read alignment 215 and determining cell trajectory 220. Examples of a computing device can include a personal computer, desktop computer laptop, server computer, a computing node within a cluster, message processors, hand-held devices, multi-processor systems, microprocessor-based or programmable consumer electronics, network PCs, minicomputers, mainframe computers, mobile telephones, PDAs, tablets, pagers, routers, switches, and the like.

FIG. 7 illustrates an example computing device 630 for implementing system and methods described in FIGs. 1-5. In some embodiments, the computing device 630 includes at least one processor 702 coupled to a chipset 704. The chipset 704 includes a memory controller hub 720 and an input/output (I/O) controller hub 722. A memory 706 and a graphics adapter 712 are coupled to the memory controller hub 720, and a display 718 is coupled to the graphics adapter 712. A storage device 708, an input interface 714, and network adapter 716 are coupled to the I/O controller hub 722. Other embodiments of the computing device 630 have different architectures.

The storage device 708 is a non-transitory computer-readable storage medium such as a hard drive, compact disk read-only memory (CD-ROM), DVD, or a solid-state memory device. The memory 706 holds instructions and data used by the processor 702. The input interface 714 is a touch-screen interface, a mouse, track ball, or other type of input interface, a keyboard, or some combination thereof, and is used to input data into the computing device 630. In some embodiments, the computing device 630 may be configured to receive input (e.g., commands) from the input interface 714 via gestures from the user. The graphics adapter 712 displays images and other information on the display 718. For example, the display 718 can show an indication of a predicted cell trajectory. The network adapter 716 couples the computing device 630 to one or more computer networks.

The computing device 630 is adapted to execute computer program modules for providing functionality described herein. As used herein, the term "module" refers to computer program logic used to provide the specified functionality. Thus, a module can be implemented in hardware, firmware, and/or software. In one embodiment, program modules are stored on the storage device 708, loaded into the memory 706, and executed by the processor 702.

The types of computing devices 630 can vary from the embodiments described herein. For example, the computing device 630 can lack some of the components described above, such as graphics adapters 712, input interface 714, and displays 718. In some embodiments, a computing device 630 can include a processor 702 for executing instructions stored on a memory 706.

The methods of aligning sequence reads and determining cell trajectories can be implemented in hardware or software, or a combination of both. In one embodiment, a non-transitory machine-readable storage medium, such as one described above, is provided, the medium comprising a data storage material encoded with machine readable data which, when using a machine programmed with instructions for using said data, is capable of displaying any of the datasets and execution and results of a cell trajectory of this invention. Such data can be used for a variety of purposes, such as patient monitoring, treatment considerations, and the like. Embodiments of the methods described above can be implemented in computer programs executing on programmable computers, comprising a processor, a data storage system (including volatile and non-volatile memory and/or storage elements), a graphics adapter, an input interface, a network adapter, at least one input device, and at least one output device. A display is coupled to the graphics adapter. Program code is applied to input data to perform the functions described above and generate output information. The output information is applied to one or more output devices, in known fashion. The computer can be, for example, a personal computer, microcomputer, or workstation of conventional design.

Each program can be implemented in a high level procedural or object oriented programming language to communicate with a computer system. However, the programs can be implemented in assembly or machine language, if desired. In any case, the language can be a compiled or interpreted language. Each such computer program is preferably stored on a storage media or device (e.g., ROM or magnetic diskette) readable by a general or special purpose programmable computer, for configuring and operating the computer when the storage media or device is read by the computer to perform the procedures described herein. The system can also be considered to be implemented as a computer-readable storage medium, configured with a computer program, where the storage medium so configured causes a computer to operate in a specific and predefined manner to perform the functions described herein.

The signature patterns and databases thereof can be provided in a variety of media to facilitate their use. "Media" refers to a manufacture that contains the signature pattern information of the present invention. The databases of the present invention can be recorded on computer readable media, e.g. any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable mediums can be used to create a manufacture comprising a recording of the present database information. "Recorded" refers to a process for storing information on computer readable medium, using any such methods as known in the art. Any convenient data storage structure can be chosen, based on the means used to access the stored information. A variety of data processor programs and formats can be used for storage, *e.g.* word processing text file, database format, etc.

### Example Kit Embodiments

Also provided herein are kits for analyzing RNA transcripts and DNA (e.g., chromatin-accessible DNA) of individual or populations of cells. The kits may include one or more of the following: fluids for forming emulsions (e.g., carrier phase, aqueous phase), barcoded beads, micro fluidic devices for processing single cells, reagents for lysing cells and releasing cell analytes, reaction mixtures for performing nucleic acid amplification reactions, and instructions for using any of the kit components according to the methods described herein.

### EXAMPLES

### Example 1: DNA Amplicons of Intron Regions - Oligo dT Priming

K-562 cells were processed using the workflow process shown in FIG. 1 using the Tapestri^{™}. In particular, single cells were partitioned into emulsions along with reagents. The reagents do not include proteases (e.g., no proteinase K) nor transposases (e.g., no transposase Tn5). The reagents included SSIV RT, 5X SSIV buffer, 10 mM dNTPs, 100 mM DTT, ribonuclease inhibitor, 50 uM oligo dT, NP-40, and dH2O. The 0.1% NP40 in the reagents cause single cells to lyse in the emulsions. The tube containing the encapsulation droplets was incubated at 55C for 10 min then 80C for 10 min. RNA and packaged DNA of single cells were processed according to the processes described in FIGs. 4A and 4B. Specifically, within the first emulsion, RNA transcripts from the single cell were primed using an oligo-dT primer and cDNA was generated using reverse transcriptase.

The cell lysate including the cDNA and packaged DNA were then emulsified in a second emulsion with a reaction mixture and a barcoded bead (with over a million barcodes releasably attached to the bead). The reaction mixture includes forward and reverse primers (with PCR handles) that target genes of interest. The forward primers are shown in Table 1. The reverse primers are shown in Table 2.

**Table 1: Forward primers (with PCR handle)**

| Gene | Sequence |
|---|---|
| CCL22 | GTACTCGCAGTAGTCTGGTTGTCCTCGTCCTCCTT |
| TNFSF4 | GTACTCGCAGTAGTCCGGTATCCTCGAATTCAAAGTATCAAAGT |
| PRF1 | GTACTCGCAGTAGTCGGTGGAGTGCCGCTTCTAC |
| IL7R | GTACTCGCAGTAGTCAGCCAATGACTTTGTGGTGACAT |
| LAG3 | GTACTCGCAGTAGTCGCGACTTTACCCTTCGACTAGA |
| HLA-A | GTACTCGCAGTAGTCCGGAGTATTGGGACCAGGAG |
| BRCA1 | GTACTCGCAGTAGTCCTGAAAGCCAGGGAGTTGGT |
| CDK1 | GTACTCGCAGTAGTCGAAGTGTGGCCAGAAGTGGA |
| NFKBIA | GTACTCGCAGTAGTCGGTGTCCTTGGGTGCTGAT |
| ITGAM | GTACTCGCAGTAGTCCGAGTACGTGCCACACCAA |
| MTOR | GTACTCGCAGTAGTCGGAAGAGGCATCTCGTTTGTACT |
| ZAP70 | GTACTCGCAGTAGTCGTGGAGAAGCTCATTGCTACGA |
| IFNG | GTACTCGCAGTAGTCTTTAAAGATGACCAGAGCATCCAAAAGA |
| CD86 | GTACTCGCAGTAGTCCACTATGGGACTGAGTAACATTCTCTT |
| CD27 | GTACTCGCAGTAGTCCTGCTCAGTGTGATCCTTGCATA |
| CXCL10 | GTACTCGCAGTAGTCTCCAGAATCGAAGGCCATCAAG |
| DDX58 | GTACTCGCAGTAGTCCCCAACCGATATCATTTCTGATCTGT |
| AKT1 | GTACTCGCAGTAGTCCCATGAGCGACGTGGCTATT |
| LAMP3 | GTACTCGCAGTAGTCGCAGTCGGGCATTCCTTCA |
| CD3E | GTACTCGCAGTAGTCAATTGTCATAGTGGACATCTGCATCA |
| CXCL1 | GTACTCGCAGTAGTCCAATCCTGCATCCCCCATAGT |
| TBX21 | GTACTCGCAGTAGTCGGATGCGCCAGGAAGTTTCA |
| CD274 | GTACTCGCAGTAGTCGTACCGCTGCATGATCAGCTA |
| IL10 | GTACTCGCAGTAGTCCCGTGGAGCAGGTGAAGAAT |
| GUSB | GTACTCGCAGTAGTCGCGAGTATGGAGCAGAAACGA |
| IL4 | GTACTCGCAGTAGTCGCACAAGCAGCTGATCCGA |
| CD28 | GTACTCGCAGTAGTCCCTCCTCCTTACCTAGACAATGAGA |
| TNFSF9 | GTACTCGCAGTAGTCCATGTTTGCGCAGCTGGT |
| FASLG | GTACTCGCAGTAGTCGCCTGTGTCTCCTTGTGATGTT |
| CA4 | GTACTCGCAGTAGTCAGGACTGCCTGCCCCATA |
| STAT3 | GTACTCGCAGTAGTCCCAATTGGAACCTGGGATCAAGT |
| IL12A | GTACTCGCAGTAGTCGAAGATGTACCAGGTGGAGTTCAA |
| CD8A | GTACTCGCAGTAGTCGAACCGAAGACGTGTTTGCAAAT |
| FOXO1 | GTACTCGCAGTAGTCTCAAGAGCGTGCCCTACTTC |
| CCR5 | GTACTCGCAGTAGTCGGCCAGAAGAGCTGAGACATC |
| MKI67 | GTACTCGCAGTAGTCCGTCGTGTCTCAAGATCTAGCTT |
| BCL2 | GTACTCGCAGTAGTCGTGGATGACTGAGTACCTGAACC |
| CCL2 | GTACTCGCAGTAGTCCGAGCTATAGAAGAATCACCAGCA |
| CD80 | GTACTCGCAGTAGTCCCAAGTGTCCATACCTCAATTTCTTTCA |
| TNFRSF4 | GTACTCGCAGTAGTCGCCCTGCACGTGGTGTAA |
| HIF1A | GTACTCGCAGTAGTCAGTACAGGATGCTTGCCAAAAGA |
| VCAM1 | GTACTCGCAGTAGTCTGCCGAGCTAAATTACACATTGATGA |
| IDO1 | GTACTCGCAGTAGTCCTAAACATCTGCCTGATCTCATAGAGT |
| CD40LG | GTACTCGCAGTAGTCGAGGCCAGCAGTAAAACAACATC |
| HLA-C | GTACTCGCAGTAGTCGAGCAGAGATACACGTGCCATAT |
| HGF | GTACTCGCAGTAGTCGGACTAACATGTTCAATGTGGGACAA |
| RORC | GTACTCGCAGTAGTCGGAAGTGGTGCTGGTTAGGA |
| PTEN | GTACTCGCAGTAGTCAGCGTGCAGATAATGACAAGGAA |
| STAT1 | GTACTCGCAGTAGTCCGATGGGCTCAGCTTTCAGA |
| CD69 | GTACTCGCAGTAGTCCGTCATGAAGGGTCCTTCCAA |
| ITGB2 | GTACTCGCAGTAGTCGCTGGGCTTCACGGACATAG |
| SAMHD1 | GTACTCGCAGTAGTCAGAGTTTGTATGCCGCAAGACA |
| KLRD1 | GTACTCGCAGTAGTCGTGAACAGAAAACTTGGAACGAAAGT |
| TLR3 | GTACTCGCAGTAGTCGGACTTTGAGGCGGGTGTTT |
| IL6 | GTACTCGCAGTAGTCAGTACCTCCAGAACAGATTTGAGAGT |
| TNFRSF14 | GTACTCGCAGTAGTCGGAGGAATGTCAGCACCAGA |
| PTGS2 | GTACTCGCAGTAGTCCCAGAGCAGGCAGATGAAATACC |
| SIT1 | GTACTCGCAGTAGTCGTGTGCTTGTGGACTCTCACA |

**Table 2: Reverse primers (with PCR handle)**

| Name | Sequence |
|---|---|
| CCL22 | gtctcgtgggctcggagatgtgtataagagacagAGTCTGAGGTCCAGTAGAAGTGTT |
| TNFSF4 | gtctcgtgggctcggagatgtgtataagagacagGAGATGAGATAAAACCCATCACAGTTGA |
| PRF1 | gtctcgtgggctcggagatgtgtataagagacagGGGTGCCGTAGTTGGAGATAA |
| IL7R | gtctcgtgggctcggagatgtgtataagagacagTGCAGGAGTGTCAGCTTTGT |
| LAG3 | gtctcgtgggctcggagatgtgtataagagacagGGGATCCAGGTGACCCAAAG |
| HLA-A | gtctcgtgggctcggagatgtgtataagagacagCCACGTCGCAGCCATACATTA |
| BRCA1 | gtctcgtgggctcggagatgtgtataagagacagCTTGTTTCACTCTCACACCCAGAT |
| CDKl | gtctcgtgggctcggagatgtgtataagagacagTCGTTTGGCTGGATCATAGATTAACATT |
| NFKBIA | gtctcgtgggctcggagatgtgtataagagacagAATAGCCCTGGTAGGTAACTCTGT |
| ITGAM | gtctcgtgggctcggagatgtgtataagagacagCATTGAATTCTTCCTGGATGCCAAA |
| MTOR | gtctc gtgggctcggagatgtgtataagagacagGCCTCCATTAAATCTCGACCATAGG |
| ZAP70 | gtctcgtggsctcggagatgtgtataagagacagGTATGTGCCCTGCTCCTTCC |
| IFNG | gtctcgtgggctcggagatgtgtataagagacagTGCTTTGCGTTGGACATTCAAG |
| CD86 | gtctcgtggsctcggagatgtgtataagagacagCTAGCTCACTCAGGCTTTGGT |
| CD27 | gtctcgtggsctcggagatgtgtataagagacagCATTGCGACAGGCACACTC |
| CXCL10 | gtctcgtgggctcggagatgtgtataagagacagTGTAGGGAAGTGATGGGAGAGG |
| DDX58 | gtctcgtggsctcggagatgtgtataagagacagTTGGGCCAGTTTTCCTTGTCT |
| AKT1 | gtctc gtgggctcggagatgtgtataagagacagCTCACGTTGGTCCACATCCT |
| LAMP3 | gtctcgtgggctcggagatgtgtataagagacagGTGTAGTCAGACGAGCACTCAT |
| CD3E | gtctcgtgggctcggagatgtgtataagagacagGTGGTGGCCTCTCCTTGTTT |
| CXCL1 | gtctcgtgggctcggagatgtgtataagagacagTGTCTCTCTTTCCTCTTCTGTTCCTA |
| TBX21 | gtctcgtgggctcggagatgtgtataagagacagCTCTGGCTCTCCGTCGTT |
| CD274 | gtctcgtgggctcggagatgtgtataagagacagGTAGCCCTCAGCCTGACAT |
| IL10 | gtctcgtggsctcggagatgtgtataagagacagTCTATAGAGTCGCCACCCTGATG |
| GUSB | gtctcntgggctcggagatgtgtataagagacagAATTCCAAATGAGCTCTCCAACCA |
| IL4 | gtctcgtggsctcggagatgtgtataagagacagCTCTCTCATGATCGTCTTTAGCCTTT |
| CD28 | gtctcgtggsctcggagatgtgtataagagacagCAAGCTATAGCAAGCCAGGACT |
| TNFSF9 | gtctcgtgggctcggagatgtgtataagagacagGCTCCTTCGTGTCCTCTTTGTAG |
| FASLG | gtctcgtggsctcggagatgtgtataagagacagGCTTCTCCAAAGATGATGCTGTGT |
| CA4 | gtctcgtgggctcggagatgtgtataagagacagACATTCCTCGATGTCCCCTTCT |
| STAT3 | gtctcgtgggctcggagatgtgtataagagacagCCATGTGATCTGACACCCTGAA |
| IL12A | gtctcgtgggctcggagatgtgtataagagacagGATTTTTGTGGCACAGTCTCACT |
| CD8A | gtctcgtgggctcggagatgtgtataagagacagGGAAGGACTTGCTCCCTCAAA |
| FOXO1 | gtctcgtgggctcggagatgtgtataagagacagGATTGAGCATCCACCAAGAACTTTT |
| CCR5 | gtctcgtgggctcggagatgtgtataagagacagGGCTGCGATTTGCTTCACA |
| MKI67 | gtctcgtgggctcggagatgtgtataagagacagTGAGTCATCTGCGGTACTGTCT |
| BCL2 | gtctcgtgggctcggagatgtgtataagagacagGGCCAAACTGAGCAGAGTCTT |
| CCL2 | gtctcgtgggctcggagatgtgtataagagacagTCTTCGGAGTTTGGGTTTGCTT |
| CD80 | gtctcntgggctcggagatgtgtataagagacagTTGTGCCAGCTCTTCAACAGA |
| TNFRSF4 | gtctcgtggsctcggagatgtgtataagagacagCAACTCCAGGCTTGTAGCTGTC |
| HIF1A | gtctcgtgggctcggagatgtgtataagagacagGGAGAAAATCAAGTCGTGCTGAAT |
| VCAM1 | gtctcgtgggctcggagatgtgtataagagacagCATGGTCACAGAGCCACCTT |
| IDO1 | gtctcgtgggctcggagatgtgtataagagacagCCCACACATATGCCATGGTGAT |
| CD40LG | gtctcgtgggctcggagatgtgtataagagacagACAGAAGGTGACTTGGGCATAGATATA |
| HLA-C | gtctcgtgggctcggagatgtgtataagagacagAGCTCCAAGGACAGCTAGGA |
| HGF | gtctcgtgggctcggagatgtgtataagagacagGAGTGGATTTCCCGTGTAGCA |
| RORC | gtctcgtgggeteggagatgtgtataagagaeagCTTAGGGAGTGGGAGAAGTCAAAG |
| PTEN | gtctcgtgggctcggagatgtgtataagagacagGATTTGACGGCTCCTCTACTGT |
| STAT1 | gtctcgtgggctcggagatgtgtataagagacagACAAAACCTCGTCCACGGAAT |
| CD69 | gtctcgtgggctcggagatgtgtataagagacagATGGCTGTCTGATGGCATTGA |
| ITGB2 | gtctcgtgggctcggagatgtgtataagagacagTTTTCCCAATGTAGCCAGTGTCA |
| SAMHD1 | gtctcgtgggctcggagatgtgtataagagacagGGCGAGTTGGATTTTGGACTGA |
| KLRD1 | gtctcgtggsctcggagatgtgtataagagacagCGGTGTGCTCCTCACTGTAA |
| TLR3 | gtctcgtgggctcggagatgtgtataagagacagTCAATAGCTTGTTGAACTGCATGATGTA |
| IL6 | gtctcgtgggctcggagatgtgtataagagacagTCAGCAGGCTGGCATTTGT |
| TNFRSF14 | gtctcgtgggctcggagatgtgtataagagacagTCACACATATGATTAGGCCAACTGT |
| PTGS2 | gtctcgtgggctcggagatgtgtataagagacagAGCTCCACAGCATCGATGTC |
| SIT1 | gtctcgtgggctcggagatgtgtataagagacagGCCAGCGAGATGAGAAATAGCA |

Chromatin accessible DNA in the packaged DNA and the cDNA are primed. Nucleic acid amplification was conducted to generate amplified nucleic acids derived from the RNA transcripts and chromatin-accessible DNA.

Amplified nucleic acids were pooled in a tube (e.g, PCR tube or Eppendorf tube) and emulsions were broken. The amplified nucleic acids underwent library preparation by adding P5 and P7 sequence adapters. Nucleic acids were sequenced to obtain sequence reads. Sequence reads were clustered according to common barcodes and aligned to a reference genome.

To verify that chromatin-accessible DNA was primed and amplified, intronic regions of DNA, as known in the reference genome, were analyzed to determine whether sequence reads corresponding to intronic regions were amplified and sequenced.

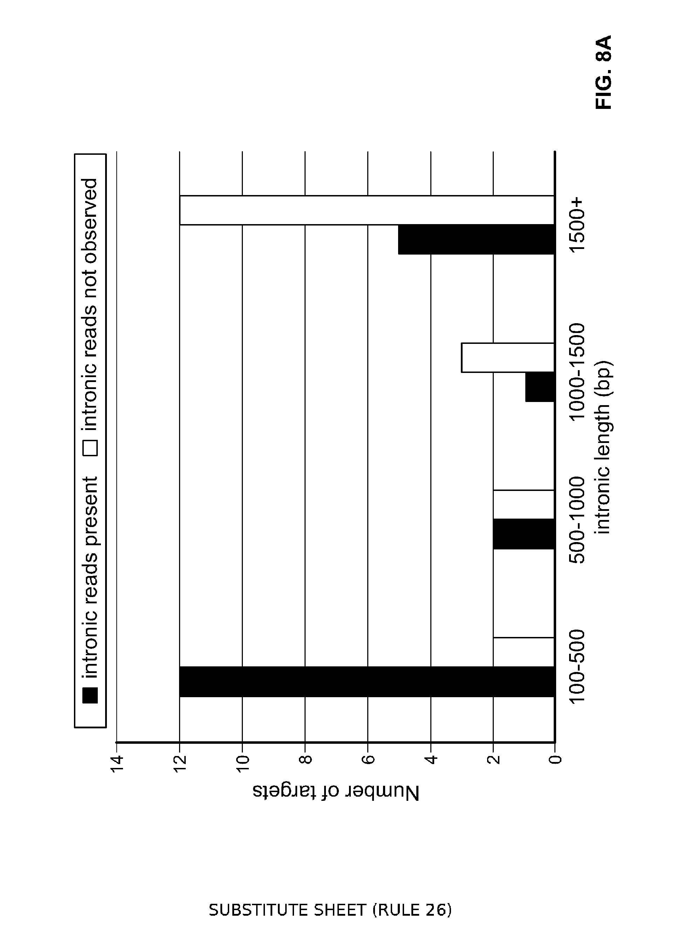

FIG. 8A depicts DNA amplicon sizes observed with reads in intronic regions obtained through oligo dT priming of K-562 cells, where no proteinase K and no transposase (Tn5) was used during encapsulation. Notably, intronic reads were present at various lengths including between 100-500 base pairs, between 500-100 base pairs, between 1000-1500 base pairs, and even beyond 1500 base pairs in length. This indicates that the corresponding genes for which these intronic reads are present are likely accessible and available for transcription. Conversely, some intronic reads were not observed. This indicates that the corresponding genes for which these intronic reads are not present are likely inaccessible and unavailable for transcription.

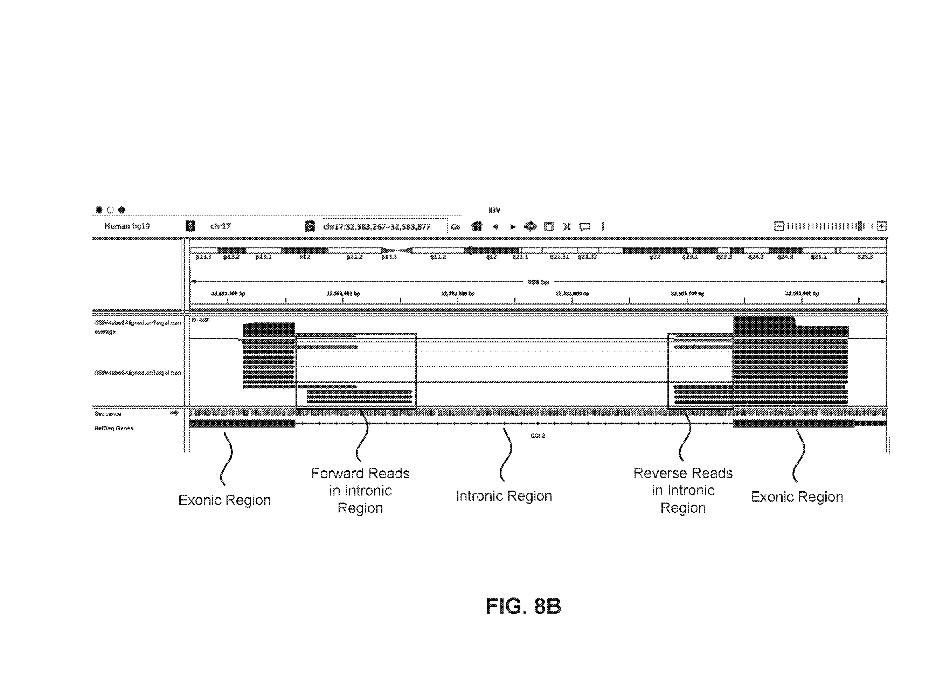

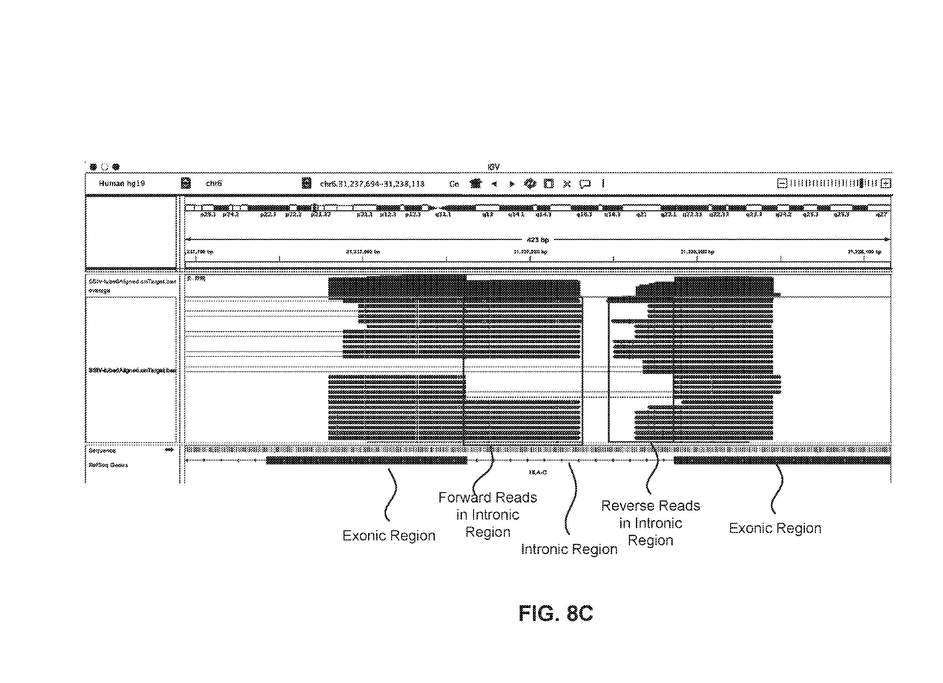

FIGs. 8B and 8C show integrative genomics viewer (IGV) screenshots of sequence reads aligned to the reference genome (aligned to the CCL2 gene and HLA-C gene, respectively). In FIGs. 8B and 8C, a number of paired reads (e.g., forward and reverse reads) align with intronic regions of the CCL2 and HLA-C. Thus, the presence of these sequence reads in the intronic regions of CCL2 and HLA-C indicate that the CCL2 and HLA-C genes are accessible and available for transcription.

Altogether, the results of FIGs. 8A-8C demonstrate that sequence reads obtained through the single-cell analysis process align with intronic regions. This indicates that chromatin-accessible DNA, when in a packaged state, was successfully accessed, primed, amplified, and sequenced.

### Example 2: DNA Amplicons of Intron Regions - Gene Specific Priming

MC7 cells were processed using the workflow process shown in FIG. 1 using the Tapestri^{™}. In particular, single cells were partitioned into emulsions along with reagents. The reagents do not include proteases (e.g., no proteinase K) nor transposases (e.g., no transposase Tn5). The reagents included SSIV RT, 5X SSIV buffer, 10 mM dNTPs, 100 mM DTT, ribonuclease inhibitor, reverse primer (34 plex), NP-40, and dH2O. The 0.1% NP40 in the reagents cause single cells to lyse in the emulsions. The tube containing the encapsulation droplets was incubated at 50°C for 10 min then 80°C for 10 min. RNA and packaged DNA of single cells were processed according to the processes described in FIGs. 4A and 4B. Specifically, within the first emulsion, RNA transcripts from the single cell were primed using reverse primers and cDNA was generated using reverse transcriptase. Reverse primers are shown in Table 3.

**Table 3- rev primers (including PCR handle)**

| Gene | Sequence |
|---|---|
| DPYD | GTCTCGTGGGCTCGGAGATGTGTATAAGAGACAGATGGAACTTGCTAAGGAA GAAAAGT |
| CASP9 | GTCTCGTGGGCTCGGAGATGTGTATAAGAGACAGGTTTTGTTTCCTGGAGGGA CC |
| UCK2 | GTCTCGTGGGCTCGGAGATGTGTATAAGAGACAGCGGGAATGGGAGACAAAG TCA |
| EPCAM | GTCTCGTGGGCTCGGAGATGTGTATAAGAGACAGATTTCAGTGTCCTTGTCTG TTCTTC |
| SOX2 | GTCTCGTGGGCTCGGAGATGTGTATAAGAGACAGTACTCTCCTCTTTGCACC CC |
| CCNA2 | GTCTCGTGGGCTCGGAGATGTGTATAAGAGACAGTTTTGGGAGAATTAAGTTT GATAGATGC |
| ABCG2 | GTCTCGTGGGCTCGGAGATGTGTATAAGAGACAGGCAGATGCCTTCTTCGTTA TGA |
| POU5F1 | GTCTCGTGGGCTCGGAGATGTGTATAAGAGACAGATATGCAAAGCAGAAACC CTCG |
| ESR1 | GTCTCGTGGGCTCGGAGATGTGTATAAGAGACAGCTCAGCATCCAACAAGGC ACT |
| TWIST1 | GTCTCGTGGGCTCGGAGATGTGTATAAGAGACAGTGGACTCCAAGATGGCAA GCT |
| SNAI2 | GTCTCGTGGGCTCGGAGATGTGTATAAGAGACAGGGCTGGCCAAACATAAGC AG |
| UCK1 | GTCTCGTGGGCTCGGAGATGTGTATAAGAGACAGATGCCTTTGATAATGATTT GATGCAC |
| MKI67 | GTCTCGTGGGCTCGGAGATGTGTATAAGAGACAGGTGTCTCAAGATCTAGCTT CTCTTC |
| VIM | GTCTCGTGGGCTCGGAGATGTGTATAAGAGACAGCTCAGGTTCAGGGAGGAA AAGT |
| CD44 | GTCTCGTGGGCTCGGAGATGTGTATAAGAGACAGCAGCCATTTGTGTTGTTGT GTG |
| FOSL1 | GTCTCGTGGGCTCGGAGATGTGTATAAGAGACAGAGTGGTTCAGCCCGAGAA CT |
| MRPL16 | GTCTCGTGGGCTCGGAGATGTGTATAAGAGACAGAAAACCCAAGCTTAGATTT ATTGAAAGG |
| MRPL21 | GTCTCGTGGGCTCGGAGATGTGTATAAGAGACAGATATGTTCCTAAAACATCC CTGAGTT |
| NEAT1 | GTCTCGTGGGCTCGGAGATGTGTATAAGAGACAGTGCCAAGCTGTCCCCTTCT C |
| KRT18 | GTCTCGTGGGCTCGGAGATGTGTATAAGAGACAGTTAATGCCTCAGAACTTTG GTGTCA |
| HMGA2 | GTCTCGTGGGCTCGGAGATGTGTATAAGAGACAGTTTTAAGGTTATGTGATTT CTCCCCA |
| NANOG | GTCTCGTGGGCTCGGAGATGTGTATAAGAGACAGCTGCGTCACACCATTGCTA T |
| CCNB2 | GTCTCGTGGGCTCGGAGATGTGTATAAGAGACAGCCATTTTCCTTGTCCTAGA ACCTT |
| CDH1 | GTCTCGTGGGCTCGGAGATGTGTATAAGAGACAGCATTGTCTGTAGCTATGAT TAGGGC |
| TK2 | GTCTCGTGGGCTCGGAGATGTGTATAAGAGACAGTGGGAAGATGCCAGAAGT GGA |
| TK1 | GTCTCGTGGGCTCGGAGATGTGTATAAGAGACAGCACAGAGTTGATGAGACG CG |
| ERBB2 | GTCTCGTGGGCTCGGAGATGTGTATAAGAGACAGTTCCGAAAGAGCTGGTCCC A |
| TYMS | GTCTCGTGGGCTCGGAGATGTGTATAAGAGACAGCGTAGCTGGCGATGTTGAA A |
| CDH2 | GTCTCGTGGGCTCGGAGATGTGTATAAGAGACAGCAGCCTCCAACTGGTATCT TCA |
| FAM32A | GTCTCGTGGGCTCGGAGATGTGTATAAGAGACAGCTTCGAAAAGCAGTCTGTA GCA |
| SNAI1 | GTCTCGTGGGCTCGGAGATGTGTATAAGAGACAGCCTTTCCCACTGTCCTCAT CTG |
| TYMP | GTCTCGTGGGCTCGGAGATGTGTATAAGAGACAGAGCCCTGTGCTCGGGAAGT |
| ABCB7 | GTCTCGTGGGCTCGGAGATGTGTATAAGAGACAGAACTAGATTTAGAATAGA AATGAACAAAGCAG |
| PCBP1 | GTCTCGTGGGCTCGGAGATGTGTATAAGAGACAGCGTGCATCATGGCAAAGT GA |

The cell lysate including the cDNA and packaged DNA were then emulsified in a second emulsion with a reaction mixture and a barcoded bead (with over a million barcodes releasably attached to the bead). The reaction mixture includes forward primers that target genes of interest. Forward primers are shown below in Table 4.

**Table 4: forward primers (including PCR handle)**

| Gene | Sequence |
|---|---|
| DPYD | GTACTCGCAGTAGTCGACCCCATCTGTTAAATTTTATAGGGC |
| CASP9 | GTACTCGCAGTAGTCCCTGGCCTTATGATGTTTTAAAGAAAAG |
| UCK2 | GTACTCGCAGTAGTCTGAGGTGGACTATCGCCAGA |
| EPCAM | GTACTCGCAGTAGTCCAGTGTACTTCAGTTGGTGCAC |
| SOX2 | GTACTCGCAGTAGTCTACCTCTTCCTCCCACTCCAG |
| CCNA2 | GTACTCGCAGTAGTCTCTCTTATTGACTGTTGTGCATGC |
| ABCG2 | GTACTCGCAGTAGTCGCTGAAGTACTGAAGCCATGACA |
| POU5F1 | GTACTCGCAGTAGTCCATAGTCGCTGCTTGATCGCT |
| ESR1 | GTACTCGCAGTAGTCGCTCCGTAAATGCTACGAAGTG |
| TWIST1 | GTACTCGCAGTAGTCTCTTCTAATTTCCAAGAAAATCTTTGGC |
| SNAI2 | GTACTCGCAGTAGTCACCTGTCTGCAAATGCTCTGTTG |
| UCK1 | GTACTCGCAGTAGTCAAGAGGCGCAGGTGGAACAT |
| MKI67 | GTACTCGCAGTAGTCTTTCTGCCATTACGTCCAGC |
| VIM | GTACTCGCAGTAGTCAATGGAAGAGAACTTTGCCGTTG |
| CD44 | GTACTCGCAGTAGTCTCTACTGTACACCCCATCCCA |
| FOSL1 | GTACTCGCAGTAGTCGCCACTCATGGTGTTGATGCT |
| MRPL16 | GTACTCGCAGTAGTCGGTCCATAGAGCGGTTGATTG |
| MRPL21 | GTACTCGCAGTAGTCCGCAAGGTCTAGTTCATTTCCAA |
| NEAT1 | GTACTCGCAGTAGTCTTGTGCTAAACGCTGGGAGG |
| KRT18 | GTACTCGCAGTAGTCCTGCTGCACCTTGAGTCAGAG |
| HMGA2 | GTACTCGCAGTAGTCACAAGTTGTTCAGAAGAAGCCTG |
| NANOG | GTACTCGCAGTAGTCGCAGAGAAGAGTGTCGCAAAA |
| CCNB2 | GTACTCGCAGTAGTCTCCCAAATCCGAGAAATGGAAAC |
| CDH1 | GTACTCGCAGTAGTCGGCCAGGAAATCACATCCTAC |
| TK2 | GTACTCGCAGTAGTCCAGCGGAATGACCTTCTCCTC |
| TK1 | GTACTCGCAGTAGTCTCGTCGATGCCTATGACAGC |
| ERBB2 | GTACTCGCAGTAGTCTTACCTATACATCTCAGCATGGCCG |
| TYMS | GTACTCGCAGTAGTCGACCAACTGCAAAGAGTGATTGA |
| CDH2 | GTACTCGCAGTAGTCCTGGTGTAAGAACTCAGGTCTGT |
| FAM32A | GTACTCGCAGTAGTCGGATCCTAAAGAAGGCATCCAAA |
| SNAI1 | GTACTCGCAGTAGTCAATCGGAAGCCTAACTACAGCGA |
| TYMP | GTACTCGCAGTAGTCAGCCTCTGACCCACGTCGA |
| ABCB7 | GTACTCGCAGTAGTCCGAGCACCATTATAGCTGTTAAACCG |
| PCBP1 | GTACTCGCAGTAGTCTCATGACCATTCCGTACCAGC |

Chromatin accessible DNA in the packaged DNA and the cDNA are primed. Nucleic acid amplification was conducted to generate amplified nucleic acids derived from the RNA transcripts and chromatin-accessible DNA.

Amplified nucleic acids were pooled in an Eppendorf tube and emulsions were broken. The amplified nucleic acids underwent library preparation by adding P5 and P7 sequence adapters. Nucleic acids were sequenced to obtain sequence reads. Sequence reads were clustered according to common barcodes and aligned to a reference genome.

To verify that chromatin-accessible DNA was primed and amplified, intronic regions of DNA, as known in the reference genome, were analyzed to determine whether sequence reads corresponding to intronic regions were amplified and sequenced.

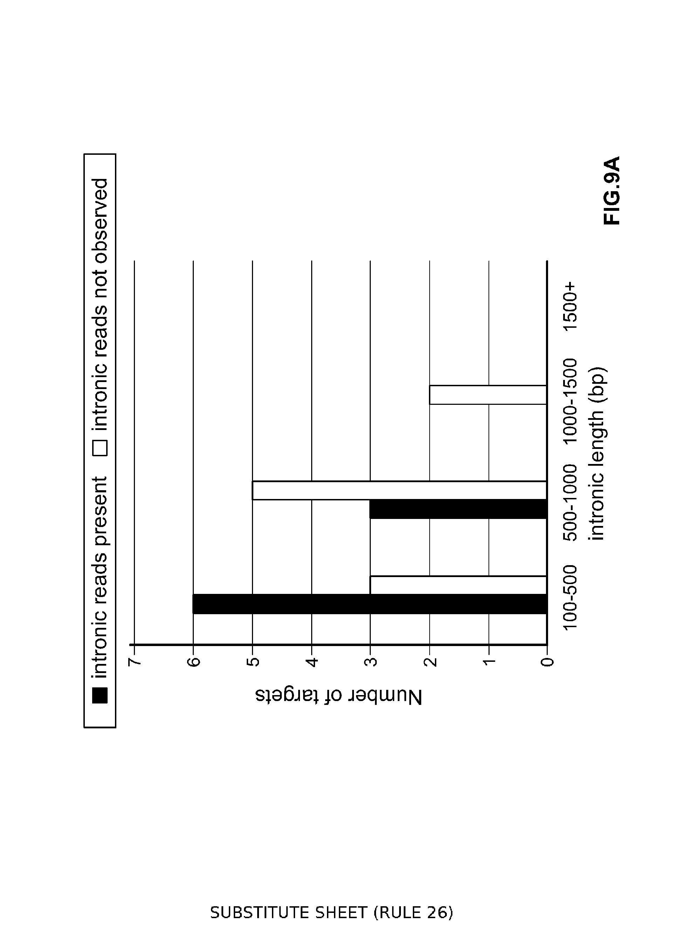

FIG. 9A depicts DNA amplicon sizes observed with reads in intronic regions obtained through gene specific priming of MCF7 cells, where no proteinase K and no transposase (Tn5) was used during encapsulation. Notably, intronic reads were present at various lengths including between 100-500 base pairs and between 500-100 base pairs. This indicates that the corresponding genes for which these intronic reads are present are likely accessible and available for transcription. Conversely, some intronic reads were not observed. This indicates that the corresponding genes for which these intronic reads are not present are likely inaccessible and unavailable for transcription.

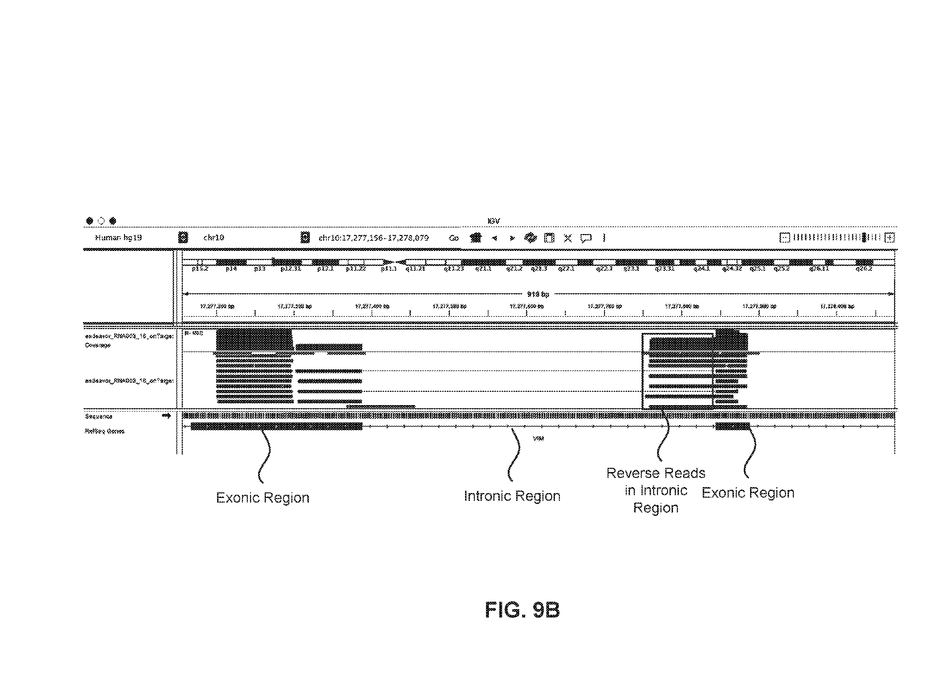

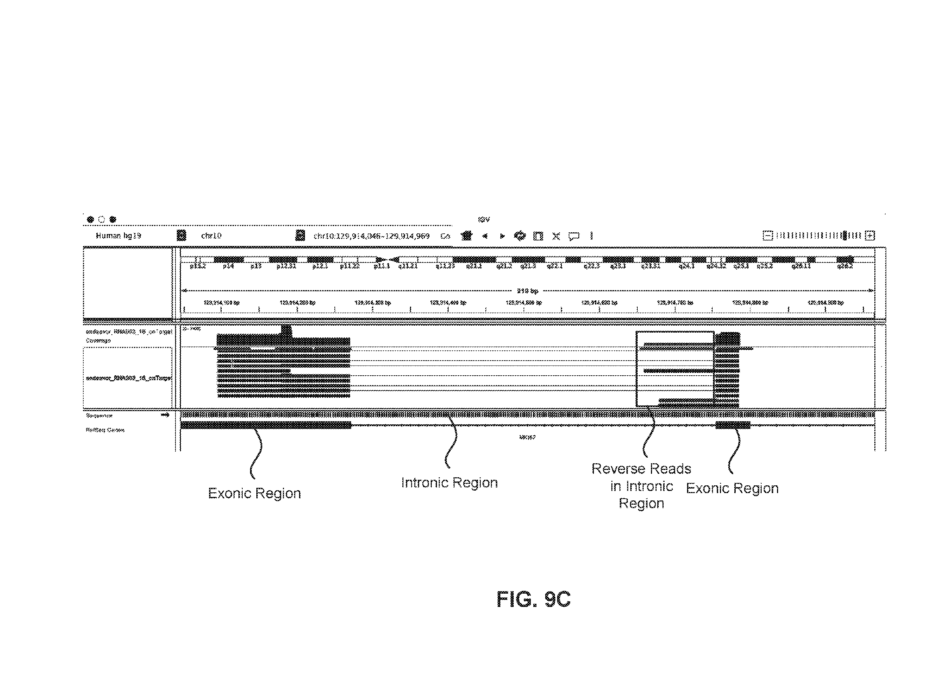

FIGs. 9B and 9C show integrative genomics viewer (IGV) screenshots of sequence reads aligned to the reference genome (aligned to the VIM gene and MKI67 gene, respectively). As shown in FIGs. 9B and 9C, reads are observed in the intronic region of the VIM gene and MK167 gene, indicating that the genes are accessible and available for transcription.

## Claims

1. A method for predicting a cell trajectory for a cell, the method comprising:
encapsulating a cell in an emulsion comprising reagents, the cell comprising at least one RNA molecule and packaged DNA comprising a segment of chromatin accessible-DNA;
lysing the cell within the emulsion, thereby exposing the RNA and the packaged DNA to the reagents, wherein the reagents comprise less than 0.50 mg/mL protease and less than 2.5% (v/v) transposase;
generating at least one cDNA molecule using the at least one RNA;
encapsulating the at least one cDNA molecule, the packaged DNA, and a reaction mixture in a second emulsion;
performing a nucleic acid amplification reaction within the second emulsion using the
reaction mixture to generate a plurality of nucleic acids,
the plurality of nucleic acids comprising:
a first nucleic acid from one of the at least one cDNA molecule; and
a second nucleic acid derived from the segment of chromatin-accessible DNA of the packaged DNA; and
sequencing the first nucleic acid and the second nucleic acid.

2. The method of claim 1, wherein the reagents
comprise less than 0.10 mg/mL protease, less than 0.01 mg/mL protease, or do not include protease; and/or
comprise less than 0.1% (v/v) transposase, less than 0.01% (v/v) transposase, or do not include transposase.

3. The method of claim 1 or 2, wherein:
performing the nucleic acid amplification reaction within the second emulsion using the reaction mixture to generate the plurality of nucleic acids comprises:
priming the segment of the chromatin-accessible DNA in the packaged DNA and
generating an extended product from the primed segment of the chromatin-accessible DNA; and/or
the method further comprises: in the emulsion, generating an extended product from a segment of the chromatin-accessible DNA in the packaged DNA, and wherein encapsulating the at least one cDNA molecule, the packaged DNA, and a reaction mixture in the second emulsion further comprises encapsulating the extended product in the second emulsion, optionally wherein generating the extended product from a segment of the chromatin-accessible DNA in the packaged DNA comprises exposing the first emulsion to a temperature between 40 °C and 60 °C, thereby destabilizing the segment of the chromatin-accessible DNA.

4. The method of any one of claims 1-3, wherein the reagents comprise a detergent for lysing the cell.

5. The method of any one of claims 1-4, wherein the reagents comprise reverse transcriptase and/or NP-40.

6. The method of any one of claims 1-5, further comprising: predicting the cell trajectory using the sequenced first nucleic acid and the sequenced second nucleic acid, optionally wherein
predicting the cell trajectory comprises using at least the sequenced first nucleic acid and second nucleic acid to determine two different states of the cell, optionally wherein the sequenced first nucleic acid is used to determine a prior state of the cell and wherein the sequenced second nucleic acid is used to determine a future state of the cell, optionally wherein the at least one RNA is
(i) previously transcribed from a DNA region that comprises one chromatin-accessible DNA, thereby indicating a commonality between the prior state and future state of the cell, or
(ii) transcribed from a DNA region that corresponds to chromatin-inaccessible DNA, thereby indicating a transition from the prior state of the cell towards the future state of the cell.

7. The method of any one of claims 1-6, wherein:
the cell trajectory is any one of a cell lineage, a cell fate, a cell function in a future state of the cell, a diseased future state of the cell, or a future cellular response to an external stimulus; and/or
the method further comprises encapsulating a first barcode and a second barcode in the second emulsion along with the at least one cDNA, at least one chromatin-accessible DNA, and the reaction mixture, optionally wherein:
the first nucleic acid comprises the first barcode; and/or
the second nucleic acid comprises the second barcode; and/or
the first barcode and second barcode share a same barcode sequence or different barcode sequences; and/or
the first barcode and second barcode are releasably attached to a bead in the second emulsion.

8. The method of any one of claims 1-7, wherein:
reverse transcribing the at least one RNA occurs within the first emulsion; and/or
the nucleic acid amplification reaction is polymerase chain reaction; and/or
the plurality of nucleic acids further comprise nucleic acids derived from other segments of chromatin-accessible DNA in the packaged DNA corresponding to intronic DNA regions, optionally wherein at least 50% of the nucleic acids derived from other chromatin-accessible DNA molecules of the packaged DNA corresponding to intronic DNA regions are between 100 to 500 base pairs in length.

9. A system comprising:
a device configured to:
encapsulate a cell in an emulsion comprising reagents, the cell comprising at least one RNA molecule and packaged DNA comprising a segment of chromatin accessible-DNA;
lyse the cell within the emulsion, thereby exposing the RNA and the packaged DNA to the reagents, wherein the reagents comprise less than 0.50 mg/mL protease and less than 2.5% (v/v) transposase;
generate at least one cDNA molecule by reverse transcribing the at least one RNA; and
encapsulate the at least one cDNA molecule, the packaged DNA, and reagents in a second emulsion;
perform a nucleic acid amplification reaction within the second emulsion to generate a plurality of nucleic acids, the plurality of nucleic acids comprising:
a first nucleic acid from one of the at least one cDNA molecule; and
a second nucleic acid derived from the segment of chromatin-accessible DNA of the packaged DNA; and
sequence the first nucleic acid and the second nucleic acid.

10. The system of claim 9, further comprising:
a computational device communicatively coupled to the device, the computational device configured to predict the cell trajectory by using the sequenced first nucleic acid and the second nucleic acid.

11. The system of claim 9 or 10, wherein the reagents:
comprise less than 0.10 mg/mL protease, less than 0.01 mg/mL protease, or do not include protease; and/or
comprise less than 0.1% (v/v) transposase, less than 0.01% (v/v) transposase, or do not include transposase.

12. The system of any one of claims 9-11, wherein:
performing the nucleic acid amplification reaction within the second emulsion using the reaction mixture to generate the plurality of nucleic acids comprises priming the segment of the chromatin-accessible DNA in the packaged DNA and generating an extended product from the primed segment of the chromatin-accessible DNA; and/or
the system further comprises: in the emulsion, generating an extended product from a segment of the chromatin-accessible DNA in the packaged DNA, and wherein encapsulating the at least one cDNA molecule, the packaged DNA, and a reaction mixture in the second emulsion further comprises encapsulating the extended product in the second emulsion, optionally wherein generating the extended product from a segment of the chromatin-accessible DNA in the packaged DNA comprises exposing the first emulsion to a temperature between 40 °C and 60 °C, thereby destabilizing the segment of the chromatin-accessible DNA.

13. .The system of any one of claims 9-12, wherein the reagents comprise a detergent for lysing the cell.

14. The system of any one of claims 9-13, wherein the reagents comprise reverse transcriptase and/or NP-40.

15. The system of any one of claims 10-14, wherein:
(i) predicting the cell trajectory comprises using at least the sequenced first nucleic acid and second nucleic acid to determine two different states of the cell, optionally wherein the sequenced first nucleic acid is used to determine a prior state of the cell and wherein the sequenced second nucleic acid is used to determine a future state of the cell, optionally wherein:
the at least one RNA is previously transcribed from a DNA region that comprises one chromatin-accessible DNA, thereby indicating a commonality between the prior state and future state of the cell, or is transcribed from a DNA region that corresponds to chromatin-inaccessible DNA, thereby indicating a transition from the prior state of the cell towards the future state of the cell; and/or
(ii) the cell trajectory is any one of a cell lineage, a cell fate, a cell function in a future state of the cell, a diseased future state of the cell, or a future cellular response to an external stimulus.

16. The system of any one of claims 9-15, wherein the device is further configured to encapsulate a first barcode and a second barcode in the second emulsion along with the at least one cDNA, at least one chromatin-accessible DNA, and the reaction mixture, optionally wherein:
the first nucleic acid comprises the first barcode; and/or
the second nucleic acid comprises the second barcode; and/or
the first barcode and second barcode share a same barcode sequence or the first barcode and second barcode share different barcode sequences; and/or
the first barcode and second barcode are releasably attached to a bead in the second emulsion.

17. The system of any one of claims 9-16, wherein:
reverse transcribing the at least one RNA occurs within the first emulsion; and/or
the nucleic acid amplification reaction is polymerase chain reaction; and/or
the plurality of nucleic acids further comprise nucleic acids derived from other segments of chromatin-accessible DNA in the packaged DNA corresponding to intronic DNA regions, optionally wherein at least 50% of the nucleic acids derived from other chromatin-accessible DNA molecules of the packaged DNA corresponding to intronic DNA regions are between 100 to 500 base pairs in length.

## Patentansprüche

1. Verfahren zum Vorhersagen einer Zelltrajektorie für eine Zelle, wobei das Verfahren Folgendes umfasst:
Einkapseln einer Zelle in einer Emulsion, die Reagenzien umfasst, wobei die Zelle mindestens ein RNA-Molekül und verpackte DNA umfassend ein Segment aus chromatinzugänglicher DNA umfasst;
Lysieren der Zelle innerhalb der Emulsion, wodurch die RNA und die verpackte DNA den Reagenzien ausgesetzt werden, wobei die Reagenzien weniger als 0,50 mg/ml Protease und weniger als 2,5 % (v/v) Transposase umfassen;
Erzeugen mindestens eines cDNA-Moleküls unter Verwendung der mindestens einen RNA;
Einkapseln des mindestens einen cDNA-Moleküls, der verpackten DNA und eines Reaktionsgemischs in einer zweiten Emulsion;
Durchführen einer Nukleinsäureamplifikationsreaktion innerhalb der zweiten Emulsion unter Verwendung des Reaktionsgemischs, um eine Vielzahl von Nukleinsäuren zu erzeugen,
wobei die Vielzahl von Nukleinsäuren Folgendes umfasst:
eine erste Nukleinsäure aus einem des mindestens einen cDNA-Moleküls; und
eine zweite Nukleinsäure, die aus dem Segment aus chromatinzugänglicher DNA der verpackten DNA abgeleitet ist; und
Sequenzieren der ersten Nukleinsäure und der zweiten Nukleinsäure.

2. Verfahren nach Anspruch 1, wobei die Reagenzien
weniger als 0,10 mg/ml Protease, weniger als 0,01 mg/ml Protease umfassen oder keine Protease beinhalten; und/oder
weniger als 0,1 % (v/v) Transposase, weniger als 0,01 % (v/v) Transposase umfassen oder keine Transposase beinhalten.

3. Verfahren nach Anspruch 1 oder 2, wobei:
das Durchführen der Nukleinsäureamplifikationsreaktion innerhalb der zweiten Emulsion unter Verwendung des Reaktionsgemischs zum Erzeugen der Vielzahl von Nukleinsäuren Folgendes umfasst: Primen des Segments der chromatinzugänglichen DNA in der verpackten DNA und Erzeugen eines erweiterten Produkts aus dem geprimten Segment der chromatinzugänglichen DNA; und/oder
das Verfahren ferner Folgendes umfasst: Erzeugen eines erweiterten Produkts, in der Emulsion, aus einem Segment der chromatinzugänglichen DNA in der verpackten DNA, und wobei das Einkapseln des mindestens einen cDNA-Moleküls, der verpackten DNA und eines Reaktionsgemischs in der zweiten Emulsion ferner das Einkapseln des erweiterten Produkts in der zweiten Emulsion umfasst, wobei optional das Erzeugen des erweiterten Produkts aus einem Segment der chromatinzugänglichen DNA in der verpackten DNA das Aussetzen der ersten Emulsion an eine Temperatur zwischen 40 °C und 60 °C umfasst, wodurch das Segment der chromatinzugänglichen DNA destabilisiert wird.

4. Verfahren nach einem der Ansprüche 1-3, wobei die Reagenzien ein Detergens zum Lysieren der Zelle umfassen.

5. Verfahren nach einem der Ansprüche 1-4, wobei die Reagenzien reverse Transkriptase und/oder NP-40 umfassen.

6. Verfahren nach einem der Ansprüche 1-5, ferner umfassend: Vorhersagen der Zelltrajektorie unter Verwendung der sequenzierten ersten Nukleinsäure und der sequenzierten zweiten Nukleinsäure, wobei optional
das Vorhersagen der Zelltrajektorie das Verwenden mindestens der sequenzierten ersten Nukleinsäure und der zweiten Nukleinsäure umfasst, um zwei verschiedene Zustände der Zelle zu bestimmen, wobei optional die sequenzierte erste Nukleinsäure verwendet wird, um einen vorherigen Zustand der Zelle zu bestimmen, und wobei die sequenzierte zweite Nukleinsäure verwendet wird, um einen zukünftigen Zustand der Zelle zu bestimmen, wobei optional die mindestens eine RNA
(i) zuvor aus einer DNA-Region transkribiert wird, die eine chromatinzugängliche DNA umfasst, wodurch eine Gemeinsamkeit zwischen dem vorherigen und dem zukünftigen Zustand der Zelle indiziert wird, oder
(ii) aus einer DNA-Region transkribiert wird, die chromatinunzugänglicher DNA entspricht, wodurch ein Übergang vom vorherigen Zustand der Zelle zum zukünftigen Zustand der Zelle indiziert wird.

7. Verfahren nach einem der Ansprüche 1-6, wobei:
die Zelltrajektorie ein beliebiges von einer Zelllinie, einem Zellschicksal, einer Zellfunktion in einem zukünftigen Zustand der Zelle, einem krankhaften zukünftigen Zustand der Zelle oder einer zukünftigen zellulären Reaktion auf einen externen Stimulus ist; und/oder
das Verfahren ferner das Einkapseln eines ersten Barcodes und eines zweiten Barcodes in der zweiten Emulsion zusammen mit der mindestens einen cDNA, mindestens einer chromatinzugänglichen DNA und dem Reaktionsgemisch umfasst, wobei optional:
die erste Nukleinsäure den ersten Barcode umfasst; und/oder
die zweite Nukleinsäure den zweiten Barcode umfasst; und/oder
sich der erste Barcode und der zweite Barcode eine identische Barcodesequenz oder unterschiedliche Barcodesequenzen teilen; und/oder
der erste Barcode und der zweite Barcode lösbar an einem Bead in der zweiten Emulsion angebracht sind.

8. Verfahren nach einem der Ansprüche 1-7, wobei:
die reverse Transkription der mindestens einen RNA innerhalb der ersten Emulsion erfolgt; und/oder
die Nukleinsäureamplifikationsreaktion eine Polymerasekettenreaktion ist; und/oder
die Vielzahl von Nukleinsäuren ferner Nukleinsäuren umfasst, die aus anderen Segmenten aus chromatinzugänglicher DNA in der verpackten DNA, die intronischen DNA-Regionen entsprechen, abgeleitet sind, wobei optional mindestens 50 % der Nukleinsäuren, die aus anderen chromatinzugänglichen DNA-Molekülen der verpackten DNA, die intronischen DNA-Regionen entsprechen, abgeleitet sind, zwischen 100 bis 500 Basenpaare lang sind.

9. System, umfassend:
eine Vorrichtung, die konfiguriert ist zum:
Einkapseln einer Zelle in einer Emulsion, die Reagenzien umfasst, wobei die Zelle mindestens ein RNA-Molekül und verpackte DNA umfassend ein Segment aus chromatinzugänglicher DNA umfasst;
Lysieren der Zelle innerhalb der Emulsion, wodurch die RNA und die verpackte DNA den Reagenzien ausgesetzt werden, wobei die Reagenzien weniger als 0,50 mg/ml Protease und weniger als 2,5 % (v/v) Transposase umfassen;
Erzeugen mindestens eines cDNA-Moleküls durch reverse Transkription der mindestens einen RNA; und
Einkapseln des mindestens einen cDNA-Moleküls, der verpackten DNA und von Reagenzien in einer zweiten Emulsion;
Durchführen einer Nukleinsäureamplifikationsreaktion innerhalb der zweiten Emulsion, um eine Vielzahl von Nukleinsäuren zu erzeugen, wobei die Vielzahl von Nukleinsäuren Folgendes umfasst:
eine erste Nukleinsäure aus einem des mindestens einen cDNA-Moleküls; und
eine zweite Nukleinsäure, die aus dem Segment aus chromatinzugänglicher DNA der verpackten DNA abgeleitet ist; und
Sequenzieren der ersten Nukleinsäure und der zweiten Nukleinsäure.

10. System nach Anspruch 9, ferner umfassend:
eine Rechenvorrichtung, die kommunikativ mit der Vorrichtung gekoppelt ist, wobei die Rechenvorrichtung dazu konfiguriert ist, die Zelltrajektorie unter Verwendung der sequenzierten ersten Nukleinsäure und der zweiten Nukleinsäure vorherzusagen.

11. System nach Anspruch 9 oder 10, wobei die Reagenzien:
weniger als 0,10 mg/ml Protease, weniger als 0,01 mg/ml Protease umfassen oder keine Protease beinhalten; und/oder
weniger als 0,1 % (v/v) Transposase, weniger als 0,01 % (v/v) Transposase umfassen oder keine Transposase beinhalten.

12. System nach einem der Ansprüche 9-11, wobei:
das Durchführen der Nukleinsäureamplifikationsreaktion innerhalb der zweiten Emulsion unter Verwendung des Reaktionsgemischs zum Erzeugen der Vielzahl von Nukleinsäuren das Primen des Segments der chromatinzugänglichen DNA in der verpackten DNA und das Erzeugen eines erweiterten Produkts aus dem geprimten Segment der chromatinzugänglichen DNA umfasst; und/oder
das System ferner Folgendes umfasst: Erzeugen eines erweiterten Produkts, in der Emulsion, aus einem Segment der chromatinzugänglichen DNA in der verpackten DNA, und wobei das Einkapseln des mindestens einen cDNA-Moleküls, der verpackten DNA und eines Reaktionsgemischs in der zweiten Emulsion ferner das Einkapseln des erweiterten Produkts in der zweiten Emulsion umfasst, wobei optional das Erzeugen des erweiterten Produkts aus einem Segment der chromatinzugänglichen DNA in der verpackten DNA das Aussetzen der ersten Emulsion an eine Temperatur zwischen 40 °C und 60 °C umfasst, wodurch das Segment der chromatinzugänglichen DNA destabilisiert wird.

13. System nach einem der Ansprüche 9-12, wobei die Reagenzien ein Detergens zum Lysieren der Zelle umfassen.

14. System nach einem der Ansprüche 9-13, wobei die Reagenzien reverse Transkriptase und/oder NP-40 umfassen.

15. System nach einem der Ansprüche 10-14, wobei:
(i) das Vorhersagen der Zelltraj ektorie das Verwenden mindestens der sequenzierten ersten Nukleinsäure und der zweiten Nukleinsäure zum Bestimmen von zwei verschiedenen Zuständen der Zelle umfasst, wobei optional die sequenzierte erste Nukleinsäure zum Bestimmen eines vorherigen Zustands der Zelle verwendet wird und wobei die sequenzierte zweite Nukleinsäure zum Bestimmen eines zukünftigen Zustands der Zelle verwendet wird, wobei optional:
die mindestens eine RNA zuvor aus einer DNA-Region transkribiert wird, die eine chromatinzugängliche DNA umfasst, wodurch eine Gemeinsamkeit zwischen dem vorherigen Zustand und dem zukünftigen Zustand der Zelle indiziert wird, oder aus einer DNA-Region transkribiert wird, die chromatinunzugänglicher DNA entspricht, wodurch ein Übergang vom vorherigen Zustand der Zelle zum zukünftigen Zustand der Zelle indiziert wird; und/oder
(ii) die Zelltrajektorie ein beliebiges von einer Zelllinie, einem Zellschicksal, einer Zellfunktion in einem zukünftigen Zustand der Zelle, einem krankhaften zukünftigen Zustand der Zelle oder einer zukünftigen zellulären Reaktion auf einen externen Stimulus ist.

16. System nach einem der Ansprüche 9-15, wobei die Vorrichtung ferner dazu konfiguriert ist, einen ersten Barcode und einen zweiten Barcode zusammen mit der mindestens einen cDNA, mindestens einer chromatinzugänglichen DNA und dem Reaktionsgemisch in der zweiten Emulsion einzukapseln, wobei optional:
die erste Nukleinsäure den ersten Barcode umfasst; und/oder
die zweite Nukleinsäure den zweiten Barcode umfasst; und/oder
sich der erste Barcode und der zweite Barcode eine identische Barcodesequenz teilen oder sich der erste Barcode und der zweite Barcode unterschiedliche Barcodesequenzen teilen; und/oder
der erste Barcode und der zweite Barcode lösbar an einem Bead in der zweiten Emulsion angebracht sind.

17. System nach einem der Ansprüche 9-16, wobei:
die reverse Transkription der mindestens einen RNA innerhalb der ersten Emulsion erfolgt; und/oder
die Nukleinsäureamplifikationsreaktion eine Polymerasekettenreaktion ist; und/oder
die Vielzahl von Nukleinsäuren ferner Nukleinsäuren umfasst, die aus anderen Segmenten aus chromatinzugänglicher DNA in der verpackten DNA, die intronischen DNA-Regionen entsprechen, abgeleitet sind, wobei optional mindestens 50 % der Nukleinsäuren, die aus anderen chromatinzugänglichen DNA-Molekülen der verpackten DNA, die intronischen DNA-Regionen entsprechen, abgeleitet sind, zwischen 100 bis 500 Basenpaare lang sind.

## Revendications

1. Méthode permettant de prédire une trajectoire cellulaire pour une cellule, la méthode comprenant :
l'encapsulation d'une cellule dans une émulsion comprenant des réactifs, la cellule comprenant au moins une molécule d'ARN et de l'ADN conditionné comprenant un segment d'ADN accessible à la chromatine ;
la lyse de la cellule au sein de l'émulsion, ce qui permet d'exposer l'ARN et l'ADN conditionné aux réactifs, lesdits réactifs comprenant moins de 0,50 mg/ml de protéase et moins de 2,5 % (v/v) de transposase ;
la génération d'au moins une molécule d'ADNc en utilisant ledit au moins un ARN ;
l'encapsulation de ladite au moins une molécule d'ADNc, dudit ADN conditionné et d'un mélange réactionnel dans une seconde émulsion ;
la réalisation d'une réaction d'amplification d'acides nucléiques au sein de la seconde émulsion en utilisant le mélange réactionnel pour générer une pluralité d'acides nucléiques,
la pluralité d'acides nucléiques comprenant :
un premier acide nucléique provenant de l'une de ladite au moins une molécule d'ADNc ; et
un second acide nucléique provenant du segment d'ADN accessible à la chromatine de l'ADN conditionné ; et
le séquençage du premier acide nucléique et du second acide nucléique.

2. Méthode selon la revendication 1, lesdits réactifs
comprenant moins de 0,10 mg/ml de protéase, moins de 0,01 mg/ml de protéase ou ne comprenant pas de protéase ; et/ou
comprenant moins de 0,1 % (v/v) de transposase, moins de 0,01 % (v/v) de transposase ou ne comprenant pas de transposase.

3. Méthode selon la revendication 1 ou 2 :
ladite réalisation de la réaction d'amplification d'acides nucléiques au sein de la seconde émulsion en utilisant le mélange réactionnel pour générer la pluralité d'acides nucléiques comprenant :
l'amorçage du segment de l'ADN accessible à la chromatine dans l'ADN conditionné et la génération d'un produit allongé à partir du segment amorcé de l'ADN accessible à la chromatine ; et/ou
ladite méthode comprenant en outre : dans l'émulsion, la génération d'un produit allongé à partir d'un segment de l'ADN accessible à la chromatine dans l'ADN conditionné, et ladite encapsulation de ladite au moins une molécule d'ADNc, de l'ADN conditionné et d'un mélange réactionnel dans la seconde émulsion comprenant en outre l'encapsulation du produit allongé dans la seconde émulsion, éventuellement ladite génération du produit allongé à partir d'un segment de l'ADN accessible à la chromatine dans l'ADN conditionné comprenant l'exposition de la première émulsion à une température comprise entre 40°C et 60°C, ce qui permet de déstabiliser le segment de l'ADN accessible à la chromatine.

4. Méthode selon l'une quelconque des revendications 1 à 3, lesdits réactifs comprenant un détergent permettant la lyse de la cellule.

5. Méthode selon l'une quelconque des revendications 1 à 4, lesdits réactifs comprenant la transcriptase inverse et/ou le NP-40.

6. Méthode selon l'une quelconque des revendications 1 à 5, comprenant en outre :
la prédiction de la trajectoire cellulaire en utilisant le premier acide nucléique séquencé et le second acide nucléique séquencé, éventuellement
ladite prédiction de la trajectoire cellulaire comprenant l'utilisation d'au moins les premier acide nucléique et second acide nucléique séquencés pour déterminer deux états différents de la cellule, éventuellement ledit premier acide nucléique séquencé étant utilisé pour déterminer un état antérieur de la cellule et ledit second acide nucléique séquencé étant utilisé pour déterminer un état futur de la cellule, éventuellement ledit au moins un ARN étant
(i) préalablement transcrit à partir d'une région d'ADN qui comprend un ADN accessible à la chromatine, ce qui permet d'indiquer un aspect commun entre l'état antérieur et l'état futur de la cellule, ou
(ii) transcrit à partir d'une région d'ADN qui correspond à de l'ADN inaccessible à la chromatine, ce qui permet d'indiquer une transition de l'état antérieur de la cellule vers l'état futur de la cellule.

7. Méthode selon l'une quelconque des revendications 1 à 6 :
ladite trajectoire cellulaire étant l'une d'une lignée cellulaire, d'un destin cellulaire, d'une fonction cellulaire dans un état futur de la cellule, d'un état futur malade de la cellule ou d'une réponse cellulaire future à un stimulus externe ; et/ou
ladite méthode comprenant en outre l'encapsulation d'un premier code à barres et d'un second code à barres dans la seconde émulsion conjointement avec ledit au moins un ADNc, au moins un ADN accessible à la chromatine et le mélange réactionnel, éventuellement :
ledit premier acide nucléique comprenant le premier code à barres ; et/ou
ledit second acide nucléique comprenant le second code à barres ; et/ou
ledit premier code à barres et ledit second code à barres partageant une même séquence de code à barres ou des séquences de code à barres différentes ; et/ou
ledit premier code à barres et ledit second code à barres étant fixés de manière amovible à une bille dans la seconde émulsion.

8. Méthode selon l'une quelconque des revendications 1 à 7 :
ladite transcription inverse dudit au moins un ARN se produisant au sein de la première émulsion ; et/ou
ladite réaction d'amplification d'acides nucléiques étant une réaction en chaîne par polymérase ; et/ou
ladite pluralité d'acides nucléiques comprenant en outre des acides nucléiques provenant d'autres segments d'ADN accessible à la chromatine dans l'ADN conditionné correspondant à des régions d'ADN introniques, éventuellement au moins 50 % des acides nucléiques provenant d'autres molécules d'ADN accessible à la chromatine de l'ADN conditionné correspondant à des régions d'ADN introniques faisant entre 100 à 500 paires de bases de long.

9. Système comprenant :
un dispositif conçu pour :
encapsuler une cellule dans une émulsion comprenant des réactifs, la cellule comprenant au moins une molécule d'ARN et de l'ADN conditionné comprenant un segment d'ADN accessible à la chromatine ;
lyser la cellule au sein de l'émulsion, ce qui permet d'exposer l'ARN et l'ADN conditionné aux réactifs, lesdits réactifs comprenant moins de 0,50 mg/ml de protéase et moins de 2,5 % (v/v) de transposase ;
générer au moins une molécule d'ADNc par transcription inverse dudit au moins un ARN ; et
encapsuler ladite au moins une molécule d'ADNc, l'ADN conditionné et les réactifs dans une seconde émulsion ;
réaliser une réaction d'amplification d'acides nucléiques au sein de la seconde émulsion pour générer une pluralité d'acides nucléiques, la pluralité d'acides nucléiques comprenant :
un premier acide nucléique provenant de l'une de ladite au moins une molécule d'ADNc ; et
un second acide nucléique provenant du segment d'ADN accessible à la chromatine de l'ADN conditionné ; et
séquencer le premier acide nucléique et le second acide nucléique.

10. Système selon la revendication 9, comprenant en outre :
un dispositif informatique couplé en communication au dispositif, le dispositif informatique étant configuré pour prévoir la trajectoire cellulaire en utilisant le premier acide nucléique séquencé et le second acide nucléique.

11. Système selon la revendication 9 ou 10, lesdits réactifs :
comprenant moins de 0,10 mg/ml de protéase, moins de 0,01 mg/ml de protéase ou ne comprenant pas de protéase ; et/ou
comprenant moins de 0,1 % (v/v) de transposase, moins de 0,01 % (v/v) de transposase ou ne comprenant pas de transposase.

12. Système selon l'une quelconque des revendications 9 à 11 :
ladite réalisation de la réaction d'amplification d'acides nucléiques au sein de la seconde émulsion en utilisant le mélange réactionnel pour générer la pluralité d'acides nucléiques comprenant l'amorçage du segment de l'ADN accessible à la chromatine dans l'ADN conditionné et la génération d'un produit allongé à partir du segment amorcé de l'ADN accessible à la chromatine ; et/ou
ledit système comprenant en outre : dans l'émulsion, la génération d'un produit allongé à partir d'un segment de l'ADN accessible à la chromatine dans l'ADN conditionné, et ladite encapsulation de ladite au moins une molécule d'ADNc, de l'ADN conditionné et d'un mélange réactionnel dans la seconde émulsion comprenant en outre l'encapsulation du produit allongé dans la seconde émulsion, éventuellement ladite génération du produit allongé à partir d'un segment de l'ADN accessible à la chromatine dans l'ADN conditionné comprenant l'exposition de la première émulsion à une température comprise entre 40°C et 60°C, ce qui permet de déstabiliser le segment de l'ADN accessible à la chromatine.

13. Système selon l'une quelconque des revendications 9 à 12, lesdits réactifs comprenant un détergent permettant la lyse de la cellule.

14. Système selon l'une quelconque des revendications 9 à 13, lesdits réactifs comprenant la transcriptase inverse et/ou le NP-40.

15. Système selon l'une quelconque des revendications 10 à 14 :
(i) ladite prédiction de la trajectoire cellulaire comprenant l'utilisation d'au moins les premier acide nucléique et second acide nucléique séquencés pour déterminer deux états différents de la cellule, éventuellement ledit premier acide nucléique séquencé étant utilisé pour déterminer un état antérieur de la cellule et ledit second acide nucléique séquencé étant utilisé pour déterminer un état futur de la cellule, éventuellement :
ledit au moins un ARN étant préalablement transcrit à partir d'une région d'ADN qui comprend un ADN accessible à la chromatine, ce qui permet d'indiquer un aspect commun entre l'état antérieur et l'état futur de la cellule, ou étant transcrit à partir d'une région d'ADN qui correspond à l'ADN inaccessible à la chromatine, ce qui permet d'indiquer une transition de l'état antérieur de la cellule vers l'état futur de la cellule ; et/ou
(ii) ladite trajectoire cellulaire étant l'une d'une lignée cellulaire, d'un destin cellulaire, d'une fonction cellulaire dans un état futur de la cellule, d'un état futur malade de la cellule ou d'une réponse cellulaire future à un stimulus externe.

16. Système selon l'une quelconque des revendications 9 à 15, ledit dispositif étant en outre conçu pour encapsuler un premier code à barres et un second code à barres dans la seconde émulsion conjointement avec ledit au moins un ADNc, au moins un ADN accessible à la chromatine et le mélange réactionnel, éventuellement :
ledit premier acide nucléique comprenant le premier code à barres ; et/ou
ledit second acide nucléique comprenant le second code à barres ; et/ou
ledit premier code à barres et ledit second code à barres partageant une même séquence de code à barres ou ledit premier code à barres et ledit second code à barres partageant des séquences de code à barres différentes ; et/ou
ledit premier code à barres et ledit second code à barres étant fixés de manière amovible à une bille dans la seconde émulsion.

17. Système selon l'une quelconque des revendications 9 à 16 :
ladite transcription inverse dudit au moins un ARN se produisant au sein de la première émulsion ; et/ou
ladite réaction d'amplification d'acides nucléiques étant une réaction en chaîne par polymérase ; et/ou
ladite pluralité d'acides nucléiques comprenant en outre des acides nucléiques provenant d'autres segments d'ADN accessible à la chromatine dans l'ADN conditionné correspondant à des régions d'ADN introniques, éventuellement au moins 50 % des acides nucléiques provenant d'autres molécules d'ADN accessible à la chromatine de l'ADN conditionné correspondant à des régions d'ADN introniques faisant entre 100 à 500 paires de bases de long.
